(19) Europäisches Patentamt
European Patent Office
Office européen des brevets

(11) EP 2 708 552 A1

(12) **EUROPEAN PATENT APPLICATION**

(43) Date of publication:
**19.03.2014 Bulletin 2014/12**

(51) Int Cl.:
***C07K 14/11*** *(2006.01)*

(21) Application number: **12184041.7**

(22) Date of filing: **12.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Medizinische Universität Wien**
**1090 Vienna (AT)**

(72) Inventors:
- **Bergmann, Michael**
  **1190 Vienna (AT)**
- **Kuznetsova, Irina**
  **1170 Vienna (AT)**

(74) Representative: **Sonn & Partner Patentanwälte**
**Riemergasse 14**
**1010 Wien (AT)**

(54) **Influenza virus**

(57) The invention discloses an isolated nucleic acid molecule comprising an influenza A virus nuclear export protein (NEP) gene wherein the codon for amino acid no. 20 of the NEP gene, glutamine, has been changed to encode for arginine, histidine, lysine, asparagine, aspartic acid, or glutamic acid, corresponding influenza A viruses and uses thereof.



Printed by Jouve, 75001 PARIS (FR)

## Description

The present invention relates to viral expression vectors.

Expression of foreign genes in viral vectors can serve a number of different medical purposes: i) it allows the generation of vaccines against pathogens, which cannot be cultivated or attenuated; ii) the use of reporter genes in viral vectors enables the generation of new diagnostic tests and screening procedures for drug development, iii) the expression of pro-inflammatory and pro-apoptotic cytokines renders oncolytic viruses more potent to counteract the tumor associated immunosuppressive microenvironment. The latter is based on the expression of pro-inflammatory and pro-apoptotic genes in the malignant tissue delivered by viral vectors, which are designed to preferentially replicate in cancer cells. Clinical phase I and II studies suggest a therapeutic benefit of local application of armed - oncolytic viruses showing complete remission in approximately 10% of the patients.

Influenza A viruses would have a number of advantages as a chimeric viral vector due to its ability to induce a strong humoral and cell-mediated immune response, the absence of any DNA intermediate life cycle stages and a ubiquitously expressed viral entry receptor. Multiple serological subtypes allow repetitive usage of the virus. Moreover, attenuated influenza A viruses have been found to be safe when applied to humans for intranasal vaccine purposes. However, the high mutation rate of influenza A viruses had significantly hampered the development of the virus as a stable chimeric vector.

A strategy to stably express high levels of a foreign protein by a replication deficient influenza A virus was described in Wolschek et al. (J. Virol. 85 (2011), 2469-2473). In this vector construct the chimeric gene replaced the ORF of a viral protein, the NS1. Stability in this vector was further supported by the fact that the first N-terminal amino acids (aa) of the transgene are shared with the NEP, an essential viral protein, which is expressed via splicing and cannot be deleted. Due to the lack of the viral IFN antagonist, the NS1 deletion vectors are highly sensitive to IFN and replication defective in IFN sensitive cells. Corresponding to the high level of attenuation, complete NS1 deletion viruses were less effective as vaccines but also less potent as oncolytic viruses when compared to attenuated influenza A viruses, in which the NS1 protein was not deleted but only truncated. It follows that an optimal influenza virus vector backbone should contain a replicating phenotype with a partial NS1 deletion. This required a modification of the expression strategy previously used for the complete NS1 deletion mutant.

It is an object of the present invention to provide new influenza virus vectors which allow a stable high expression of a heterologous protein. It is a further object to provide improved influenza viruses and pharmaceutical compositions containing such viruses for vaccination purposes or other treatments involving influenza viruses.

Therefore, the present invention provides an isolated nucleic acid molecule comprising an influenza A virus nuclear export protein (NEP) gene wherein the codon for amino acid no. 20 of the NEP gene, glutamine, has been changed to encode for arginine, histidine, lysine, asparagine, aspartic acid, or glutamic acid.

With the present invention, a new variant of influenza virus protein NEP is provided which enables a significant improvement, especially in expression technology for heterologous proteins (expression of transgenes with an influenza expression system) as well as in influenza vaccination strategies.

Changing Gln20 in the NEP results in a most stable replicating influenza A virus vector expressing high levels of whole proteins, including secreted proteins. The resulting viral vectors according to the present invention are specifically suited for a variety of purposes, especially as

- oncolytic viral vectors expressing a transgene in a tumor microenvironment,
- antigenic vectors to induce an immunity against pathogens,
- tool for diagnostic testing, and
- genetic stable vaccine backbone viruses.

Stability of the present vectors has been shown with the present invention in production cell lines, such as Vero cells or B16f1 cells as well as in an experimental mouse model. In a therapeutical set-up it has been shown that IL-2 can be stably expressed in a murine B16 tumor model leading to increased survival.

The term "isolated" refers to a purified in vitro preparation, isolation and/or purification of a nucleic acid molecule such as a vector, plasmid or a virus according to the present invention, so that it is not associated with in vivo substances, or is substantially purified from in vitro substances. An isolated virus preparation is generally obtained by in vitro culture and propagation and is substantially free from other infectious agents. As used herein, "substantially free" means below the level of detection for a particular infectious agent using standard detection methods for that agent. A "recombinant" virus is one which has been manipulated in vitro, e.g., using recombinant DNA techniques, to introduce changes to the viral genome, or otherwise artificially generated.

The "influenza A virus" is an enveloped negative-strand virus with eight RNA segments encapsulated with nucleoprotein (NP). The eight single-stranded negative-sense viral RNAs (vRNAs) encode for eleven proteins (HA, NA, NP, M1, M2, NS1, NEP, PA, PB1, PB1-F2, PB2). The total genome size is 13,588 bases. The segmented nature of the genome allows for the exchange of entire genes between different viral strains during cellular cohabitation. The eight RNA segments are:

- HA encodes hemagglutinin (about 500 molecules of hemagglutinin are needed to make one virion). The extent of infection into host organism is determined by HA. Influenza viruses bud from the apical surface of polarized epithelial cells (e.g. bronchial epithelial cells) into lumen of lungs and are therefore usually pneumotropic. The reason is that HA is cleaved by tryptase clara which is restricted to lungs. However HAs of H5 and H7 pantropic avian viruses subtypes can be cleaved by furin and subtilisin-type enzymes, allowing the virus to grow in other organs than lungs;
- NA encodes neuraminidase (about 100 molecules of neuraminidase are needed to make one virion).
- NP encodes nucleoprotein;
- M encodes two matrix proteins (the M1 and the M2) by using different reading frames from the same RNA segment (about 3000 matrix protein molecules are needed to make one virion);
- NS encodes two distinct non-structural proteins (NS1 and NEP) by using different reading frames from the same RNA segment;
- PA encodes an RNA polymerase;
- PB1 encodes an RNA polymerase and PB1-F2 protein (induces apoptosis) by using different reading frames from the same RNA segment;
- PB2 encodes an RNA polymerase.

The genome segments have common terminal sequences, and the ends of the RNA strands are partially complementary, allowing them to bond to each other by hydrogen bonds. After transcription from negative-sense to positive-sense RNA the +RNA strands get the cellular 5' cap added by cap snatching, which involves the viral protein NS1 binding to the cellular pre-mRNAs. The cap is then cleaved from the cellular pre-mRNA using a second viral protein, PA. The short oligo cap is then added to the influenza +RNA strands, allowing its processing as messenger RNA by ribosomes. The +RNA strands also serve for synthesis of - RNA strands for new virions.

The RNA synthesis and its assembly with the nucleoprotein takes place in the cell nucleus, the synthesis of proteins takes place in the cytoplasm. The assembled virion cores leave the nucleus and migrate towards the cell membrane, with patches of viral transmembrane proteins (hemagglutinin, neuraminidase and M2 proteins) and an underlying layer of the M1 protein, and bud through these patches, releasing finished enveloped viruses into the extracellular fluid. The influenza virus life cycle begins with binding of the hemagglutinin (HA) to sialic acid-containing receptors on the surface of the host cell, followed by receptor-mediated endocytosis. The low pH in late endosomes triggers a conformational shift in the HA, thereby exposing the N-terminus of the HA2 subunit (the so-called fusion peptide). The fusion peptide initiates the fusion of the viral and endosomal membrane, and the matrix protein (Ml) and RNP complexes are released into the cytoplasm. RNPs consist of the nucleoprotein (NP), which encapsidates vRNA, and the viral polymerase complex, which is formed by the PA, PB1, and PB2 proteins. RNPs are transported into the nucleus, where transcription and replication take place.

Although influenza B and C viruses are structurally and functionally similar to influenza A virus, there are some differences, however, the present invention is also applicable for influenza B and C, because codon for amino acid no. 20 of the NEP gene of influenza A virus, glutamine, corresponds to codon for amino acid no. 18 of the NEP gene of influenza B virus, alanine, and codon for amino acid no. 104 of the NEP (NES) gene of influenza C virus, serine. Changes of Ala18 of influenza B NEP to Glu, Asp, Val are specifically preferred; alanine replacement can also be effected by Asn, Gln, Ser, Arg, Lys or His. Changes of Ser104 of influenza C NEP (NES) to Arg, Ser, Lys, Asn, Thr, Ile, Arg are specifically preferred; serine replacement can also be effected by Glu, Gln, Ser, Arg, Asp, Val, Lys or His. For the present invention, the exchange always referred to is the Gln20 exchange for influenza A, except explicitly stated otherwise (i.e. explicit referral to influenza B or C).

The similarities of influenza A to influenza B and C allowed transformation of the present invention to influenza B and C, however, there are, as already stated, also differences between these influenza viruses which have to be considered when adapting the present invention to influenza B and C. For example, the M segment of influenza B virus encodes two proteins, Ml and BM2, through a termination-reinitiation scheme of tandem cistrons, and the NA segment encodes the NA and NB proteins from a bicistronic mRNA. Influenza C virus, which has 7 vRNA segments, relies on spliced transcripts to produce Ml protein; the product of the unspliced mRNA is proteo lyrically cleaved to yield the CM2 protein. In addition, influenza C virus encodes a HA-esterase (HEF) rather than individual HA and NA proteins. There is no significant differences between influenza A and B and C with respect to NEP.

The open reading frames of influenza virus gene segments are known in the art or can readily be determined using standard molecular biology and virology techniques. In particular, influenza virus NS gene segments and the open reading frames of the NSl and NEP proteins encoded by such segments are known in the art or can readily be determined. For example and not by limitation, the influenza virus A/WSN/33 (WSN) NS gene segment can be found in GenBank (GenBank No. Z21498; GI: 296585). The open reading frame for the WSN NSl is from nucleotides 27 to 719. The open reading frame for the WSN NS2 is from nucleotides 27 to 56 of exon 1 and nucleotides 529 to 864. The influenza virus A/Puerto Rico/8/34 (PR8) NS gene segment can be found in GenBank (e.g., GenBank No. AF389122.1 GI21693177). The open reading frame for the PR8 NSI is from nucleotides 27 to 719. The open reading frame for the PR8 NEP (otherwise known as NS2) is from 27 to 56 of exon 1 and nucleotides 529 to 864. In specific embodiments, either the NSI ORF, the NEP ORF or both are further codon optimized (without changing the protein sequence) to, e.g., avoid repetitive sequences, to increase protein expression and/or to increase the stability of the NS gene segment. Techniques for codon optimization are known in the art, e.g. from WO 2011/044561 A1).

The NS2 (NEP) protein of influenza A virus contains a highly conserved nuclear export signal (NES) motif in its amino-terminal region ($_{12}$ILMRMSKMQL$_{21}$, A/WSN/33), which is thought to be required for nuclear export of viral ribonucleoprotein complexes (vRNPs) mediated by a cellular export factor, CRM1. Within NES amino acids are distributed with certain spacing ψXXXψXXψXψ, where ψ indicates important hydrophobic residues such as leucine, isoleucine, methionine, valine, or phenylalanine.

Following viral mutants were described: I12C/L13V, I12L, S17C, K18R/M19F, K18S/M19V, M14Y, M16L, M16A, S17C, M19A, L21A and Q20L. All mutants have a general feature - a delay in nuclear export of vRNPs in comparison with wild-type virus. No difference in expression levels of NEP among the NS2 mutants was observed. The influenza virus with the mutation in position Q20L (Iwatsuki-Horimoto et al., J. Virol. 78 (2004), 10149-10155) had a peak titer at least 2 log lower than wt on MDCK, and had a delay in nuclear export of vRNP. This shows that this mutant is not suitable for protein expression or therapeutic uses.

None of introduced mutation could completely prevent viral replication. Amino acid mutations I12C/L13V, I12L and S17C did not have any influence on the viral growth that indicates that just one of hydrophobic residue at position 12 or 13 is sufficient for optimal function of NEP. All other viral constructions containing mutation in one of the hydrophobic residues M14, M16, M19 or L21 had a reduction in the viral titer. Changing methionine at position 16 or 19 to another hydrophobic residue (M16L, M19V or M16F) affected viral growth. Changing methionine at position 16 or 19 and leucine at position 21 to alanine leads to absence of the yield of infectious virus. Thus, methionine at position 16 or 19 and the leucine at the position 21 are crucial for viral replication.

Although, it was also shown that mutation of methionine at position 16 of NEP to isoleucine plays a role in host adaptation of human H5N1 isolate (A/Thailand/1(KAN-1)/2004) by compensation the inefficient adaptation of the polymerase.

Mutation in NEP according to the present invention (and exemplified by the NS-116-GFP/A virus Q20R in the example section) was never described before in the way of its connection with stable expression of transgene by viral vectors.

Any influenza virus NEP gene may be modified to produce a modified influenza virus NEP gene segment as described herein. [In one embodiment, the modified influenza virus NEP (NS) gene segment described herein is derived from an influenza A virus. In another embodiment, the modified influenza virus gene segment described herein is derived from an influenza B virus. In another embodiment, the modified influenza virus gene segment described herein is derived from an influenza C virus.] In certain embodiments, the modified influenza virus NEP gene or NS gene segment is a chimera of two influenza virus types, subtypes or strains. For example, the modified influenza virus NS gene segment may comprise the open reading frame of NSI from influenza A virus and the open reading frame of NEP from an influenza B virus. As another example, the modified influenza virus NS gene segment may comprise the open reading frame of NSI from one influenza A virus strain and the open reading of NEP from a different influenza A virus strain.

Examples of influenza A viruses include subtype H10N4, subtype H10N5, subtype H10N7, subtype H10N8, subtype H10N9, subtype HI 1NI, subtype HI 1N13, subtype HI 1N2, subtype HI 1N4, subtype HI 1N6, subtype HI 1N8, subtype HI 1N9, subtype H12N1, subtype H12N4, subtype H12N5, subtype H12N8, subtype H13N2, subtype H13N3, subtype H13N6, subtype H13N7, subtype H14N5, subtype H14N6, subtype H15N8, subtype H15N9, subtype H16N3, subtype HINI, subtype H1N2, subtype H1N3, subtype H1N6, subtype H1N9, subtype H2N1, subtype H2N2, subtype H2N3, subtype H2N5, subtype H2N7, subtype H2N8, subtype H2N9, subtype H3N1, subtype H3N2, subtype H3N3, subtype H3N4, subtype H3N5, subtype H3N6, subtype H3N8, subtype H3N9, subtype H4N1, subtype H4N2, subtype H4N3, subtype H4N4, subtype H4N5, subtype H4N6, subtype H4N8, subtype H4N9, subtype H5N1, subtype H5N2, subtype H5N3, subtype H5N4, subtype H5N6, subtype H5N7, subtype H5N8, subtype H5N9, subtype H6N1, subtype H6N2, subtype H6N3, subtype H6N4, subtype H6N5, subtype H6N6, subtype H6N7, subtype H6N8, subtype H6N9, subtype H7N1, subtype H7N2, subtype H7N3, subtype H7N4, subtype H7N5, subtype H7N7, subtype H7N8, subtype H7N9, subtype H8N4, subtype H8N5, subtype H9N1, subtype H9N2, subtype H9N3, subtype H9N5, subtype H9N6, subtype H9N7, subtype H9N8, and subtype H9N9. Specifically preferred subtypes are H1N1, H1N2, H3N2 and H5N1.

Specific examples of strains of influenza A virus are disclosed in paragraphs [0099] and [0100] of WO 2011/014504 A1, especially Influenza A/PR8/8/34 virus.

According to the present invention, the codon for amino acid no. 20 of the NEP gene, glutamine, has been changed in the nucleic acid molecule according to the present invention to encode for arginine, histidine, lysine, asparagine, aspartic acid, glutamic acid. Especially replacement of glutamine at position 20 by arginine has proven to result in an excellently stable influenza virus vector allowing a high expression level of heterologous proteins.

The nuclear export signal of the NEP, which spans for position 12 to 21 interacts with the PB2 protein. Changes within the interacting sequence of the proteins lead to virus stability. An example is the generated NS1-116/GFP/A virus, which contains a mutation in NEP at position 20 and a mutation in the PB2 at position 535 as compared to an unstable counterpart. The combination of the mutation according to the present invention (Gln20 of NEP) with such changes within the PB2 signal sequence, especially at position 535 of PB2, is therefore a preferred embodiment of the present invention (especially a Met535Ile exchange).

Preferably, the nucleic acid molecule according to the present invention comprises further coding sequences, specifically further influenza sequences, such as HA, NA, NP, M1, M2, NS1, PA, PB1, PB1-F2 and/or PB2 sequences. It is especially preferred that the nucleic acid molecule according to the present invention further comprises an influenza NS1 gene.

The nucleic acid encoding the NEP variant according to the present invention has turned out to be specifically suited for expressing heterologous proteins in an influenza expression system. Accordingly, it is preferred that the nucleic acid according to the present invention further comprises sequences which allow expression of foreign ("heterologous", "transgene") proteins, especially an expression promoter, a terminator sequence, a heterologous gene for expression, siRNA, short regulatory nucleotide/RNA sequences, or combinations thereof.

Influenza virus vectors have been shown to be good expression systems for such foreign proteins. Preferably, the heterologous gene for expression is located 5' to the NEP gene. According to a preferred embodiment, the heterologous gene for expression preferably encodes for a therapeutic protein, especially a vaccination antigen, or a cytokine, especially IL-2, IL-24, IL-15, IL-12, GM-CSF, Antigens, TBc Antigens, or Luciferase.

The nucleic acid molecules according to the present invention may also be provided in virus-like particles or in compositions comprising further nucleic acids encoding for the other influenza nucleic acids, e.g. preferably as a set of expression vectors capable of directly expressing in culturing cells genomic influenza vRNA segments to provide the complete genomic vRNA segments of an influenza virus (disclosed e.g. in WO 2001/004333 A1 or WO 2003/072725 A2).

According to another aspect, the present invention relates to an influenza A virus comprising an influenza A virus nuclear export protein (NEP) gene wherein the codon for amino acid no. 20 of the NEP gene, glutamine, has been changed to encode for arginine, histidine, lysine, asparagine, aspartic acid, or glutamic acid. Again, it is preferred that the codon for amino acid no. 20 of the NEP gene, glutamine, has been changed to encode for arginine.

The virus can be provided in any form, such as whole virus, inactivated or intact viruses, or (live) attenuated viruses. Live, attenuated influenza virus vaccines, can also be used for preventing or treating influenza virus infection, according to known method steps. Attenuation is achieved in a single step by transfer of attenuated genes from an attenuated donor virus to a replicated isolate or reassorted virus according to known methods; however, it is preferred to use multiple attenuating mutations to prevent reversion to a virulent phenotype. Since resistance to influenza A virus is mediated by the development of an immune response to the HA and NA glycoproteins, the genes coding for these surface antigens must come from the circulating wild-type strains. The attenuated genes are derived from the attenuated parent. Preferably, genes that confer attenuation preferably do not code for the HA and NA glycoproteins. Otherwise, these genes could not be transferred to reassortants bearing the surface antigens of the clinical virus isolate.

Many donor viruses have been evaluated for their ability to reproducibly attenuate influenza viruses. As a non-limiting example, the A/ Ann Arbor(AA)/6/60 (H2N2) cold adapted (ca) donor virus can be used for attenuated vaccine production. Reassortant progeny are then selected at 25°C (restrictive for replication of virulent virus), in the presence of an H2N2 antiserum, which inhibits replication of the viruses bearing the surface antigens of the attenuated A/AA/6/60 (H2N2) ca donor virus. A large series of H1N1 and H3N2 reassortants have been evaluated in humans and found to be satisfactorily: (a) infectious, (b) attenuated for seronegative children and immunologically primed adults, (c) immunogenic and (d) genetically stable. The immunogenicity of the ca reassortants parallels their level of replication. Thus, the acquisition of the six transferable genes of the ca donor virus by new wild-type viruses has reproducibly attenuated these viruses for use in vaccinating susceptible adults and children.

Other attenuating mutations can be introduced into influenza virus genes by site-directed mutagenesis to rescue infectious viruses bearing these mutant genes. Attenuating mutations can be introduced into non-coding regions of the genome, as well as into coding regions. Such attenuating mutations can also be introduced, for example, into the PB2 polymerase gene or the NS gene or by codon optimisation (WO 2011/044561 A1). Thus, new donor viruses can also be generated bearing attenuating mutations introduced by site-directed mutagenesis, and such new donor viruses can be used in the production of live attenuated reassortant H1N1 and H3N2 vaccine candidates in a manner analogous to that described above for the A/AA/6/60 ca donor virus. It is preferred that such attenuated viruses maintain the genes from the virus that encode antigenic determinants substantially similar to those of the original clinical isolates. This is because the purpose of the attenuated vaccine is to provide substantially the same antigenicity as the original clinical isolate of the virus, while at the same time lacking infectivity to the degree that the vaccine causes minimal change of inducing a serious pathogenic condition in the vaccinated mammal.

To construct a replicating competent attenuated viral vector, which depicts high growth in cancer cells and stably expresses a transgene the viral property of adaptation for the purpose of viral engineering was exploited by the present invention. Previously obtained low GFP-expressing influenza virus vector was optimized by continued selecting procedure targeting the most bright fluorescent plaques in tumor cells. As a result a viral vector was obtained, which could stably express high levels of GFP in several tumor cell lines and in vivo. Genetic analysis revealed that optimization with respect to stability was mainly dependent on a single change in NEP Q20, especially Q20R. Importantly, it was shown that GFP-insert is dispensable and could be replaced by another transgene like human IL-2 without affecting efficiency of the optimized vector.

The virus can thus be attenuated or inactivated, formulated and administered, according to known methods, as a vaccine to induce an immune response in an animal, e.g., a mammal. Methods are well-known in the art for determining whether such attenuated or inactivated vaccines have maintained similar antigenicity to that of the clinical isolate or high growth strain derived therefrom. Such known methods include the use of antisera or antibodies to eliminate viruses expressing antigenic determinants of the donor virus; chemical selection (e.g., amantadine or rimantadine); HA and NA activity and inhibition; and DNA screening (such as probe hybridization or PCR) to confirm that donor genes encoding the antigenic determinants (e.g., HA or NA genes) are not present in the attenuated viruses.

The viruses or nucleic acids according to the present invention are preferably provided as pharmaceutical compositions. Such compositions of the present invention are suitable for inoculation or for parenteral, nasal or oral administration, and comprise attenuated or inactivated influenza viruses or nucleic acids according to the present invention, optionally further comprising sterile aqueous or non-aqueous solutions, suspensions, and emulsions. The compositions can further comprise auxiliary agents or excipients, as known in the art. These compositions according to the present invention are generally presented in the form of individual doses (unit doses).

According to a preferred embodiment, the present influenza A virus further comprises codon changes in the genes compared to the influenza virus A/PR/8/34 (H1N1)(sequences provided in Table 4), preferably in the NS1 gene, the PB2 gene, the HA gene or combinations thereof, especially wherein

- the codon for amino acid no. 368 of the NS1 gene, tyrosine, has been changed to encode for a different amino acid at this position in the NS1 gene,
- the codon for amino acid no. 535 of the PB2 gene, methionine, has been changed to encode for a different amino acid at this position in the PB2 gene,
- the codon for amino acid no. 479 of the HA gene, glycine, has been changed to encode for a different amino acid at this position in the HA gene,
- or combinations thereof.

Other changes relate to attenuation mutations, such as those disclosed in WO 2011/044561 A1, WO 2008/156778 A2, WO 2008/048306 A2, WO 2012/088428 A1 (as examples for a large number of attenuation mutations).

Preferably, the influenza A virus according to the present invention further comprises the following codon changes:

- the codon for amino acid no. 368 of the NS1 gene, tyrosine, has been changed to encode for histidine at this position in the NS1 gene,
- the codon for amino acid no. 535 of the PB2 gene, methionine, has been changed to encode for isoleucine at this position in the PB2 gene, and
- the codon for amino acid no. 479 of the HA gene, glycine, has been changed to encode for a arginine at this position in the HA gene.

According to a preferred embodiment of the present invention, the influenza A virus further comprises a heterologous gene for expression, preferably encoding for a therapeutic protein, especially a vaccination antigen, or a cytokine (e.g. IL-24, IL-15, IL-12, GM-CSF, Antigens, TBc Antigens, or Luciferase).

As already stated above, a preferred use of the molecules and compositions according the present invention is the provision of influenza vaccine. The isolated nucleic acid or influenza A virus according to the present invention are preferably provided for use as a vaccine.

The present invention therefore provides a vaccine composition for inducing a protective immune response in a subject comprising any of the influenza viruses described herein and a pharmaceutically acceptable carrier.

It should be understood that an influenza virus, especially an attenuated virus, according to the present invention, where used to elicit a protective immune response in a subject or to prevent a subject from becoming afflicted with a virus-associated disease, is administered to the subject in the form of a composition additionally comprising a pharmaceutically acceptable carrier. Pharmaceutically acceptable carriers are well known to those skilled in the art and include, but are not limited to, one or more of 0.01-0.1 M and preferably 0.05 M phosphate buffer, phosphate-buffered saline (PBS), or 0.9 % saline. Such carriers also include aqueous or non-aqueous solutions, suspensions, and emulsions.

Aqueous carriers include water, alcoholic/aqueous solutions, emulsions or suspensions, saline and buffered media. Examples of non-aqueous solvents are propylene glycol, polyethylene glycol, vegetable oils such as olive oil, and injectable organic esters such as ethyl oleate. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Solid compositions may comprise nontoxic solid carriers such as, for example, glucose, sucrose, mannitol, sorbitol, lactose, starch, magnesium stearate, cellulose or cellulose derivatives, sodium carbonate and magnesium carbonate. For administration in an aerosol, such as for pulmonary and/or intranasal delivery, an agent or composition is preferably formulated with a nontoxic surfactant, for example, esters or partial esters of $C_6$ to $C_{22}$ fatty acids or natural glycerides, and a propellant. Additional carriers such as lecithin may be included to facilitate intranasal delivery. Pharmaceutically acceptable carriers can further comprise minor amounts of auxiliary substances such as wetting or emulsifying agents, preservatives and other additives, such as, for example, antimicrobials, antioxidants and chelating agents, which enhance the shelf life and/or effectiveness of the active ingredients. The instant compositions can, as is well known in the art, be formulated so as to provide quick, sustained or delayed release of the active ingredient after administration to a subject. Certain embodiments of any of the instant immunization and therapeutic methods further comprise administering to the subject at least one adjuvant. An "adjuvant" shall mean any agent suitable for enhancing the immunogenicity of an antigen and boosting an immune response in a subject. Numerous adjuvants, including particulate adjuvants, suitable for use with both protein- and nucleic acid-based vaccines, and methods of combining adjuvants with antigens, are well known to those skilled in the art. Suitable adjuvants for nucleic acid based vaccines include, but are not limited to, Quil A, imiquimod, resiquimod, and interleukin-12 delivered in purified protein or nucleic acid form. Adjuvants suitable for use with protein immunization include, but are not limited to, alum, Freund's incomplete adjuvant (FIA), saponin, Quil A, and QS-21.

On the other hand, the isolated nucleic acid or influenza A virus according to the present invention are provided for use in oncolytic tumour therapy. Examples for such uses are disclosed e.g. in WO 2008/140621 A2.

Another aspect of the present invention relates to the use of an isolated nucleic acid or an influenza A virus according to the present invention for the expression of a heterologous gene. The present mutation provided in the NEP gene allows for stable vectors for high expression of heterologous proteins.

Further, the present invention relates to the use of an isolated nucleic acid or an influenza A virus according to the present invention for diagnosis in virology. The influenza virus according to the present invention is specifically suitable in a diagnostic testing system as disclosed by Rimmelzwaan et al. (Vaccine 29 (2011), 3424-3430) as alternative to the hemagglutinin inhibition (HI) assay. The HI assay always requires a second assay for the detection of residual virus replication, which makes it laborious to perform and less suitable for high throughput testing of large numbers of samples. Rimmelzwaan et al., 2011, have disclosed an alternative method for the detection of human serum antibodies, which is based on the use of reporter viruses that express the green fluorescent protein (GFP) upon infection of target cells. GFP-expressing viruses were generated carrying the HA of a variety of antigenically distinct H5N1 influenza viruses. This method proved easy to perform and can be carried out rapidly. The influenza virus according to the present invention is an attractive alternative for the classical virus neutralization assay and suitable for large sero-epidemiological studies or for the assessment of vaccine efficacy in clinical trials.

According to another aspect, the present invention relates to an isolated nucleic acid molecule comprising an influenza B or influenza C virus nuclear export protein (NEP) gene wherein the codon for amino acid no. 18 of the NEP gene of influenza B, alanine, has been changed, or wherein the codon for amino acid no. 104 of the NEP gene of influenza C, serine, has been changed. The present invention which has been exemplified above for influenza A is also applicable for influenza B and C. The disclosure above and the embodiments disclosed can therefore also be adapted as disclosed for influenza A virus. Preferred amino acid substitutions for influenza B and C have been disclosed above; the use of the nucleic acids comprising NEP genes for influenza B and C according to the present invention (i.e. with substitutions of Ala18 and Ser104, respectively, can be practised as disclosed for the Gln20 substitutions disclosed herein for influenza A NEP.

The invention is further described by the following examples, yet without being restricted thereto.

Fig. 1 shows growth of NS-116-GFP/O and NS-116-GFP/A viruses on Vero (a) and B16F1 (b) cells. Subconfluent monolayers of cells were infected with NS-116-GFP/O NS-116-GFP/A viruses at a multiplicity of infection (MOI) 0.01. At indicated time points supernatant was collected and amount of virus particles was determined by 50% tissue culture infective dose ($TCID_{50}$) assay.

Fig. 2 shows a schematic representation of recombinant NS gene structure expressing GFP. The light grey box represents 12 random amino acids fused to GFP, the dark grey - 29 amino acids encoding sequence derived from the initial GFP cloning vector.

Fig. 3 shows GFP expression of cells infected with NS-116-GFP/O and NS-116-GFP/A viruses (a). Fluorescent GFP expression in B16F1 cells, which were infected for 48 hours with virus at MOI 0.01. Type of virus is indicated at the top. (b) Genetical stability of NS-116-GFP/O and NS-116-GFP/A viruses on Vero and B16F1. Subconfluent monolayer of cells was infected with NS-116-GFP/O NS-116-GFP/A viruses at a MOI 0.01. 72 hours after infection supernatants were collected, diluted in a ration 1:100 in OptiPro medium and a new cell monolayer was infected. After 5 passages a number of GFP -positive plaques was determined as a per cent from the total amount of plaques in plaque assay presented as means ± standard error of the mean (SEM) from three independent experiments.

Fig. 4 shows growth kinetic of adapted-change-revertant viruses in Vero (a) and B16F1 (b) cells. Subconfluent monolayers of cells were infected with the viruses at a multiplicity of infection (MOI) 0.01. At indicated time points supernatant was collected and amount of virus particles was determined by 50% tissue culture infective dose assay on Vero cells data were presented as means ± SEM.

Fig. 5 shows mean fluorescence intensity of B16f1 cells infected with GFP-expressing viruses. Subconfluent monolayers of cells were infected with the viruses at a MOI 1. 24 hours post infection cells were collected and fluorescence intensity was measured by flow cytometry. Data is presented as means ± SEM.

Fig. 6 shows viral replication and genetic stability of NS-116-GFP/O (a) and NS-116-GFP/A (b) in mice. Balb/c mice were infected intranasal under narcosis with $1 \times 10^6$ PFU/animal of virus either with NS1-116-GFP/O or NS-116-GFP/A. On days 2, 4 and 6 viruses in mouse lungs were titrated using a limiting dilution assay on Vero cells. Titers ($TCID_{50}$) were determined by estimating cytopathic effect (CPE) and fluorescence. Data represent means ± SEM.

Fig. 7 shows schematic representation of recombinant NS gene structure expressing human IL-2. The middle grey box represents amino acids encoding an autoproteolytic 2A cleavage site, the light grey box - a modifed mouse IgK-derived signal peptide.

Fig. 8 shows replication kinetic of parental NS-116-GFP/A and modified NS-116-IL-2/A virus on B16f1 (A) cells and Vero (B) cells. Cells were infected at a MOI 0.01. Viral titers in the supernatants were assessed at 12, 24, 36, 48, 60 and 72 hours post infection and presented as means ±SEM.

Fig. 9 shows stability of the IL-2 transgene of chimeric NS-116-IL-2 virus. Stability was assessed after 2 and 5 passages NS-116-IL-2/A virus in Vero cells (a) Reverse-transcription-PCR analysis of chimeric NS/IL2 segment in virus NS-116-IL-2. Viral RNA was isolated from supernatant infected cells 72 hours post infection. cDNA was synthesized using Uni12 primer and was used as a templates for PCR. Control was presented by pHW-plasmid coding NS-116-IL-2 (pl) . mr.....marker, pl... plasmid DNA, 2... NS-116-IL-2 passaged 2 times, 5.... NS-116-IL2 passage 5 times (b) Total IL-2 level in supernatants of passaged NS-116-IL2 infected Vero cells. Cells were infected with virus at MOI 1, supernatant was collected 24 hours post-infection and the level of IL-2 production was determined by ELISA. IL-2 levels are given as a mean ± SEM. Nr of passages of NS-116-IL2 virus before IL-2 was determined as indicated on the left: the 5th, 5 passages, the 2nd, two passages.

Fig 10 shows biological activity of IL-2 expressed by NS-116-IL-2/A virus. Monolayer of Vero cells was infected with NS-116-GFP/A or NS-116-IL-2/A viruses and supernatants were collected after 24 hours. Concentration of IL-2 in supernatant was determined with ELISA. $1 \times 10^6$ T lymphocytes from healthy donors were labelled with CSFE and incubated 120 hours with supernatants. Proliferation of cells was measured by flow cytometry and data were presented as means ± SEM.

Fig. 11 shows the effect of NS-116-GFP/A and NS-116-GFP/A/IL-2 treatment on the survival of B16f1 melanoma-bearing mice. Seven 6-week-old C57/BL6 mice were injected in the right groin with $1 \times 10^5$ B16f1 cells. On day 5 and 7 after tumor implantation mice were given an intratumoral injection of $1 \times 10^7$ $TCID_{50}$ /mouse either NS-116-GFP/A or NS-116-GFP/A/IL-2 virus. On day 11 and 14 after tumor implantation mice were treated with $1 \times 10^8$ $TCID_{50}$ /mouse of either NS-116-GFP/A or NS-116-GFP/A/IL-2 virus. Mice were killed when tumor volumes reached 2000 $mm^3$.

Fig. 12 shows the principle of the modification of HA cleavage site influenza A virus for oncolytic tumor therapy.

Fig. 13 shows Vero cells infected with an AT-GFP virus in co-cultivation system with human neutrophils. A - AT virus, B - AT virus + neut. elastase, C - AT virus + trypsin, D - AT virus +neutrophils (Fig. 13a); Vero cells infected with an AE-GFP virus in co-cultivation system with human neutrophils. A - AE virus, B - AE virus + neut. elastase, C - AE virus+trypsin, D - AE virus + neutrophils (Fig. 13b).

Fig. 14 shows the titers of AT and AE on Vero cells in co-cultivation system with human neutrophils.

Fig. 15 shows the therapeutic effect of oncolytic influenza partial NS1 deletions virus containing an elastase cleavage site (AE) as compared to corresponding virus containing the wild-type trypsin on Panc-1 cells. Tumor cells were inoculated into the hindflank of nude mice. Viruses were injected at the same site 5 times in 24 hour intervals starting at day 7. Mice were culled, when tumor reached a volume of 600 $mm^3$. x axis: days after tumor cell injection. y axis: survival. diamonds: saline treatment (control), squares: partial deletion virus containing a trypsin cleavage site (AT), triangle: partial deletion virus containing an elastase cleavage site (AE) (virus corresponding to Fluvastrix) .

EXAMPLES:

1. Self-optimisation of Influenza virus vector leads to stabilisation of transgene expression

The development of chimeric viral vectors enables alternative vaccine strategies, new diagnostic tools in virology and the concept of armed virotherapy to combat cancer. The usage of influenza A virus vectors for this purpose has been hampered by the instable nature of the virus, leading to rapid loss of the transgene in replicating vaccine viruses. In the following example the influenza virus inherent property of self-optimization was used to select a mouse melanoma cell - adapted vector (NS-116/A virus), stably expressing a green fluorescent protein (GFP) sequence fused to truncated viral NS1 protein. Although adaptation was associated with the appearance of 4 coding mutations in the viral genome, stability of the transgene expression was primarily dependent on a single mutation of Q20 in the viral nuclear export protein (NEP). Adaptation was correlated with lower vector-induced expression of IL-6 in infected cells. Importantly, being adapted to B16f1 murine melanoma cells the selected vector has acquired stability in other cell types and *in vivo* in mouse lungs. Moreover, when the GFP-reporter insert was exchanged to another foreign sequence such as human interleukin-2 (IL-2) the stable high expression of the transgene was retained. Using the IL-2-expressing influenza NS-116/A virus vector for oncolytic tumor therapy allowed for complete remission in 25% of B16f1 tumor-bearing mice.

Materials and methods

Cell lines

Murine melanoma cell line B16F1 was maintained in DMEM-Ham's F12 medium (GIBCO, Invitrogen, Carlsbad, Calif) supplemented with 10 % heat inactivated fetal calf serum (FCS; GIBCO) and 2mM Glutamax-I (GIBCO). Monkey kidney epithelial cells Vero adapted to grow in serum-free medium were maintained in serum-free OPTIPRO medium (GIBCO). All cell lines were incubated at 37°C in humidified atmosphere of 5%$CO_2$.

Viruses

The influenza A/Puerto Rico/8/34/NS1-125GFP was kindly provided by C. Kittle (Institute of Applied Microbiology, University of Natural Resources and Applied Life Sciences, Vienna, Austria). The virus was generated on the base of A/PR/8/34 (H1N1) strain where NS genomic segment was modified by inserting GFP open reading frame after nucleotide position 400. The virus had truncated 125 N-terminal amino acids NS1 protein and contained intact sequences essential for splicing of NEP mRNA. This virus was adapted by serial passages to the growth on B16F1 in presence of 5 μg/μl trypsin (Sigma-Aldrich, Vienna, Austria). For propagation of all viruses Vero cells were infected at a multiplicity of infection (m.o.i.) of 0.1 and incubated in OPTIPRO medium containing 5 mg/ml trypsin at 37°C for 2 days. Virus titers were determined by plaque assay on Vero cells or by a 50% tissue culture infective dose ($TCID_{50}$) It should be noted that corresponding to the parental NS-GFPStSt virus the NS-116-GFP are influenza A/PR8/34-like viruses but do contain 22 amino acids changes in comparison with the A/PR8/34 wild type sequence.

Construction of plasmids

Viral RNA of NS1-116-GFP/O and NS-116-GFP/A viruses were isolated from 300 μl of supernatant of infected cells using the RNeasy kit according to the manufacturer's instruction (Qiagen, Hilden, Germany). cDNAs were synthesized using Uni12 primer (5' - AGCAAAAGCAGG-3') and Superscript II reverse transcriptase (Promega, Madison, Wi, USA) according to the manufacturer's protocol. Then cDNAs were amplified by PCR using segment-specific primers and nucleotide sequence analysis of PCR-products was performed (GeneArt-Invitrogen, Regensburg, Germany). All eight cDNA segments of NS-116-GFP/A virus were individually cloned into the bidirectional plasmids pHW2000 (GeneArt) containing the RNA polymerase I (pol I)-RNA polymerase II (pol II) system (Hoffmann et al., PNAS 97 (2000), 6108-6113).

Generation of viruses by reverse genetic

$1\times10^6$ Vero cells were transfected with 0.5 μg of 8 plasmid coding proteins of NS-116-GFP/A virus using the Nucleofector technique (Amaxa-Lonza, Visp, Switzerland), according to Amaxa® Cell Line Nucleofector® Kit V. To generate NS-116-IL-2/A, NS-116-GFP/A/NEP20, NS-116-GFP/A/NS368, NS-116-GFP/A/PB2 535, NS-116-GFP/A/PB2 535 NS368, NS-116-GFP/A/PB2 535 NEP20 viruses appropriate pHW-NS-116 plasmids coding modified PB2 and NS segments were used. For generation of NS-116-GFP/A/HA NC virus plasmid coding HA of *A/New Caledonia/20/99* have been used. Next 96 hours after transfection cells were examined for GFP expression and presence of cytopathic effect (cpe). When cpe reached 70% supernatant was collected and viral offspring were passaged twice in Vero cells and virus titer of the stock was determined by plaque assay.

Plaque assay and 50% tissue culture infective dose ($TCID_{50}$) assay

For plaque assay Vero cells were seeded in six-well plates in concentration $1\times10^6$/well. 24 hours later medium was removed, ten-fold dilutions of virus were prepared in OPTIPRO medium and 150 μl of each dilution was added to the cells and incubated 45 min at the room temperature with the frequent shaking. After that, inoculums were removed and cells were overlayed with OPTIPRO medium, containing 0.6% of agar (Sigma-Aldrich), 0.01 % DEAE-Dextran (Sigma-Aldrich), and 5 mg/ml trypsin (Sigma-Aldrich). Plates were incubated at 37C° in humidified atmosphere of 5% $CO_2$ and 48 hours later number of plaques was counted and results were expressed as a $\log_{10}$ plaque form units (PFU) per ml.

For $TCID_{50}$ assay Vero cells were seeded in ninety six-well plates in concentration $3\times10^6$ cells/plate. Next day medium was removed and cells were incubated 45 min with 50 μl of 10 fold virus dilution in OPTIPRO medium. After incubation 50 μl of OPTIPRO medium containing 10 mg/ml trypsin were added to each well. Virus titer was calculated 48 hours later by the method of Reed and Muench method and presented as a $\log_{10}$ $TCID_{50}$ per ml.

Determination of multicycle virus replication

To evaluate virus propagation in indicated cell lines, subconfluent monolayer of the cells was infected with relevant virus at a multiplicity of infection 0.01 and supernatant was collected 6 times post infection with the interval 12 hours. The virus titers were determined by $TCID_{50}$ assay and plaque assay. All experiments were independently repeated 3 times and the results were presented as a mean ± standard deviation.

Genetic stability of the viruses

B16F1 and Vero cells were seeded on 12 well plates with the concentration $0{,}4\times10^6$ cells per well and next day were infected with appropriate viruses at a MOI 0.01. After 45 min of incubation at the room temperature, viral inoculum was removed and cells were overlayed with 1.5 ml of OPTIPRO medium containing 5 mg/ml of trypsin. 72 hours post infection supernatant was collected, diluted in a ratio 1:100 with OPTIPRO medium and a new monolayer either of B16f1 or Vero cells was infected. This procedure was repeated 5 times. After that plague assay was performed and number of GFP-positive and GFP-negative plaques was counted.

After the serial passages of NS-116- IL-2/A virus viral RNA was isolated from supernatant infected cells and cDNA of NS-116-IL2/A gene was synthesized as described above. Amplification of the fragment was performed using PCR with the sense primer Len - 134 5'-AGCAAAAGCAGGGTGACAAAG-3' and the antisense primer - NS843 5'-CTCTTGTTCCACTTCAAAT-3'. pHW2000 -NS-116-IL-2 plasmid was used a positive control. PCR products were separated by electrophoresis in 1% agarose gel, visualized by Gel-Red (Biotium, Hayward, CA, USA) and molecular weights were compared.

Virus replication and stability in mouse lungs

6-week old Balb/c mice were infected intranasally with $1\times10^6$ PFU/animal of NS-116-GFP/A virus or NS-116-GFP/O virus under ether anesthesia. On day 2, 4 and 6 mice were sacrificed; the lungs were aseptically removed and homogenized in 1 ml of OPTIPRO medium with a rotor homogenizer. The virus yield in homogenates was determined by $TCID_{50}$ assay in Vero cells with evaluation of the presence cpe and GFP expression.

Enzyme-linked immunosorbent assay.

The absolute amount of IL-2 produced by Vero cells after infection with NS-116- IL-2/A was measured by Ready-Set-Go! Human Interleukin-2 kit (eBioscience, Vienna, Austria) according the manufacture's protocol. $1\times10^6$ cell were seeded in each well of six-well plate, infected next day with the 2$^{nd}$ and the 5$^{th}$ passages of NS-116-IL-2/A virus or NS-116-GFP/A (stock virus) as a control at a MOI 1 and incubated 2 hours at 37°C. Then virus inoculum was removed, cells were washed twice with PBS and 2 ml of OPTIPRO medium containing 10% of FCS were added to the each well with the following incubation over 24 hours. The results presented as a mean from three independent experiments ± standard deviation.

Biological activity of IL-2 expressed by NS-116- IL-2/A virus Isolation of $CD3^+$ lymphocytes from healthy donors

Peripheral blood mononuclear cells (PBMCs) from the healthy donors were isolated by using Ficoll-Plaque Plus (GE Healthcare, Bio-Science AB, Uppsala, Sweden) density gradient centrifugation. Monoculture of $CD3^+$ cells was depleted from PMBCs by magnetic selection over LS-positive separation MACS columns using an anti-CD3 antibody labeled with magnetic beads (Miltenyi Biotech, Auburn, Calif, USA).

CSFE labeling.

$CD3^+$ cells were stained with CSFE according the manufacturer's protocol of CellTrance CSFE cells proliferation kit (Molecular Probes, Eugene, Or, USA). Briefly, $CD3^+$ lymphocytes were washed twice with PBS and were incubated in concentration $1\times10^6$/ml 30 min in prewarmed (37°C) PBC containing 10μM CSFE. After this period, cells were washed twice, resuspended in OPTIPRO medium containing 10% FCS and placed on 24 well plate in concentration $1\times10^6$/well.

IL-2 treatment and flow cytometry analysis.

After CSFE-labeling $CD3^+$ cells were stimulated with IL-2. Therefore appropriate volume of supernatant infected with NS-116-IL-2/A Vero cells were added to the cell suspension to reach the end concentration 5, 50 or 150 ng/ml and cells were cultured next 6 days. As a positive control human recombinant IL-2 (Sigma-Aldrich) was used. Lymphocytes were then stained with mAbs to cell surface antigens - PE/Cy7 conjugated anti-CD3 (BioLegend, San Diego, CA, USA), PC-5 conjugated anti-CD8 (Backman Coulter, Fullerton, CA, USA) and ApC/Cy7 conjugated anti-CD4 (BioLegend). Flow cytomentry analysis was performed for fractionation of proliferating cells in different T-cells subtypes by using Gallios flow pyrometer (Backman Coulter).

In vivo treatment of established tumor.

B16f1 cells - $5\times10^5$ in 100 μl were injected subcutaneously into the right groin of female C57BL/6 mice. Administration of therapeutic viral injections was began on day 5, when tumors were visible and were ~ 1-2 mm in diameter. Tumor size were measured using digital calipers each second day and tumor volume (V) was calculated using the formula $V = \frac{\pi b a^2}{6}$, where ***a*** **-** the smallest diameter, ***b*** - the perpendicular diameter.

Statistical analysis

Statistical analysis was performed by two-tailed Student t-test and Kaplan-Meyer survival curves were compared using the log-rank (Mantel-Cox) test (Prism 5, Graph Pad software, CA, USA).

Results

Two-step selection of virus with high and stable GFP expression

In order to obtain an influenza A virus, which stably expresses high levels of a foreign protein a previously constructed chimeric virus NS1-GFPStSt (Kittel et al., J. Virol. 79 (2005), 10672-10677) was optimized. The expression of GFP by this vector was at the border of the detection limit. However, appearance of single plaques with bright fluorescence at a frequency of around 1:1000 was noticed. Thus, the first optimization step was done by selecting such naturally occurring high GFP expressing variants in Vero cells. 10 rounds of plaque purification gave rise to the viral mutant NS116-GFP/O which could induce 50 % of bright fluorescence plaques and which could grow up to 7 log of a 50% tissue culture infective dose ($TCID_{50}$)/ml (Fig. 1a) in Vero cells. Sequence analysis of this virus revealed a deletion in the NS1 sequence, upstream of the GFP insert, resulting in a frame shift and loss of the stop codon leading to the formation of an NS1-GFP fusion reading frame. The new virus NS-116-GFP/O contained 104 N-terminal aa of NS1 protein fused to GFP via a stretch of 12 random amino acids derived from frame 2 two of the NS segment (Fig.2). Moreover, the NS1-GFP fusion protein contained 29 foreign amino acids at its carboxyl-end. Those sequences were derived from the multiple cloning site of the initial plasmid vector. The virus NS-116-GFP/O displayed only limited growth of 4.5 log of a $TCID_{50}$/ml (Fig 1a) and weak fluorescence on B16f1 mouse melanoma cells (Fig.1b).

In order to generate a high GFP-expressing virus, which also grew well in tumor cells a second optimization step was performed. This consisted of multiple rounds of plaque purification of the NS-116-GFP/O virus in B16f1 murine melanoma cells, always selecting the largest fluorescent plaques. After 15 rounds of plaque purifications the resultant passaged virus vector NS-116-GFP/A had started to grow to a titer of 7 log $TCID_{50}$/ml (Fig.1a). This enhanced growth of this "adapted" virus correlated with uniform bright fluorescence of the infected B16f1 cells, which was not observed for the "original" NS-116-GFP/O virus (Fig.1b) . In order to analyze the stability of the GFP expression in the two different chimeric vectors the number of GFP positive plaques was determined and compared it with the total number of plaques after 5 consecutive passages of each virus in B16f1 cells (n=6). Infection of B16f1 and in Vero cells with the NS-116-GFP/O virus resulted in only 42,7%±9% and 16,7%±2,4% of GFP-positive plaques, respectively (Fig. 3a). This indicates an instable transgene expression in both cell lines. In contrast, the adapted vector NS-116-GFP/A could form 96.5% ± 1.7% and 97.6% ± 1.4% GFP-positive plaques in B16f1 and Vero cell, respectively (Fig. 3b). Thus, surprisingly, the plaque purification step in B16f1 cells not only resulted in an enhanced growth but importantly led to a vector with a stable transgene expression.

Genetic changes in NS-116-GFP/A virus

Next, the genetic differences between the non-B16f1-adpated, instable "original "NS-116-GFP/O virus and the stable "adapted" NS-116-GFP/A viruswere determined. Total genome sequencing of the virus NS-116-GFP/A revealed differences in only four nucleotides in PB2, HA and NS segments, which all resulted in changes of amino acids (Table 1). Interestingly, one mutation found within the NS1-GFP protein was located in the C-terminal artificial 29 amino acid tail. All segments of the NS-116-GFP/A virus were cloned into the bidirectional plasmids pHW2000 (Hoffmann et al., PNAS 97 (2000), 6108-6113), which were subsequently used to generate a complete synthetic NS-116-GFP/A virus. This virus had identical properties to the naturally selected variant with respect to viral growth and GFP expression, providing further evidence that no other changes would account for the observed different viral phenotype.

Analysis of genetic changes on viral phenotypes in vitro

To understand which of the genetic changes between the two viruses account for the difference in viral phenotype each acquired mutation was reverted in PB2 or NS segments of the NS-116-GFP/A virus. The role of the HA mutation was tested by exchanging the whole PR8-derived HA segment to the *A/New Caledonia/20/99* (H1N1) HA gene. Rescued viruses are presented in Table 2. A virus with the combination of two repaired mutations in PB2 (I535M) and in NEP (Q20R) could not be rescued suggesting an inappropriate growth of this mutant.

First the growth of rescued mutants was analyzed. Viruses NS-116-GFP/A/NS368 and NS-116-GFP/A/NEP20, which contain single reverted mutations in NS segment reverted (NS1 H368Y and NEP R20Q, respectively) showed significantly decreased viral titers in both, Vero and B16f1 cells in comparison with NS-116-GFP/A virus (Table 1 and Fig. 4). The I535M reversion of the mutation in PB2 gene did not affect viral growth neither on Vero nor B16f1 cells. Interestingly, the NS-116/A/PB2 535 NS368 virus, which contained with two repaired mutations (I535M in PB2 and H368Y in NS1) could grow to the titers similar to NS-116-GFP/A virus. This could indicate that the combination of all mutations rather than single changes accounts for growth differences between NS-116-GFP/O and NS-116-GFP/A. The exchange of the whole HA segment of the influenza A/PR8/34 virus (H1N1) to the influenza *A/New Caledonia/20/99* also led to a significant reduction in growth as compared to NS-116-GFP/A virus. This difference in growth might also be explained by the host range restriction of the exchange of the HA.

To study the influence of mutations on genetic stability of the transgene, single- and double-change reversion-viruses were analyzed for the ratio of GFP positive and negative plaques after 5 passages in Vero and B16 cells. Surprisingly, only reversion of the mutation in NEP (mutant virus NS116-GFP/A/NEP20) led to an instable GFP expression (Table 2). For all the other revertants the stability of transgene expression was not significantly different as compared to the NS116-GFP/A, although a tendency of instability was observed for the NS-116-GFP/A/PB2 535 isolate (Table 2). The same, exchange of HA segment did not have any effect on vector stability. Thus, growth capacity of constructed viruses was not necessarily correlated with the stability of transgene expression.

Next the intensity of GFP fluorescence of mutant viruses in B16f1 cells was determined. Highest GFP brightness was observed for NS-116-GFP/A/PB2 535 and NS-116-GFP/A (Fig. 5). Thus, growth or stability was not necessarily directly correlated with fluorescence intensity, but the latter correlated with mutations in the NS segment. This might be explained by cis acting signals in NS segment on RNA or protein misfolding of the reporter.

To better understand the enhanced growth and or stability of the NS-116-GFP/A virus as compared to the NS-116-GFP/O possible differences with respect to the induction of the innate immune system in B16f1 cells were determined in B16f1 cells. Minimal and not significant different concentrations of virus induced TNF, murine IFNalpha, IL-10 and MCP-1 were observed. In contrast, NS-116-GFP/O induced high levels of IL-6, whereas NS-116-GFP/A did not ($p < 0.01$). Analysing IL-6 levels of single and double revertant viruses (Table 3) indicated a direct correlation of virus growth with the induction of IL-6 ($p < 0,05$) It should be noted, that IL-6 levels induced by influenza A/PR/8/34 wild type virus were undetectable, whereas a replication deficient influenza A/PR/8/34 virus with impaired NS1 (delNS1 virus) function induced concentrations of IL-6 comparable to the NS-116-GFP/NEP virus in this assay. Thus, adaptation of a previously poor growing virus to better growth seems to be associated with diminished levels of IL-6.

Growth and genetic stability *in vivo*

Next, it was investigated whether NS-116-GFP/A vector has growth and genetic stability advantages *in vivo.* Infection of mice revealed that both original and adapted viruses could grow in mouse lungs up to 6 logs and were cleared at day 6 after infection. In order to assess the genetic stability *in vivo* the TCID50/ml titers were compared according to fluorescence or cytopathic effect. In case of NS116-GFP/O virus, the titer based on GFP assessment was significantly reduced on day 4 and absent on day 6, whereas the yield of GFP negative virus particles was detected at each time point (Fig. 6a). This indicates a loss of the transgene in vivo for this virus. In contrast, the overall titer and titer of GFP positive particles was the same on all days examined for the NS116-GFP/A virus (Fig. 6b). Thus, NS-116-GFP/A virus appears to be genetically stable *in vivo.*

Generation and in vitro-characterization of a NS-116-IL-2 A virus

Next it was investigated, whether the vector backbone of the NS-116-GFP/A virus would also allow stable expression of another foreign protein, such as IL-2. In virus NS-116-IL-2/A the sequence of GFP was replaced by the ORF of human mature IL-2. The IL-2 gene was preceded by an autoproteolytic 2A cleavage site and a modified mouse IgK-derived signal peptide allowing secretion of the cytokine (Fig. 7). The virus NS-116-IL-2/A was rescued in Vero cells.

Growth analysis of NS-116-IL-2/A virus indicated that the replacing of the GFP sequence in the NS-116-GFP/A virus by IL-2 did not significantly change the viral titer neither on B16f1 (Fig 8a) cells nor on Vero cells (Fig. 8b). Moreover, substituting of GFP to IL-2 did not affect the genetic stability of the vector. RT-PCR analysis using NS-specific primers indicated that the NS-116-IL-2/A chimeric segment was retaining during at least 5 serial passages in B16f1 cells (data not shown) or Vero cells (Fig.9a).

Infection of Vero cells with IL-2 expressing vector allowed accumulation of 192 $\pm$ 20ng/ml of IL-2 (n=3) in the tissue culture supernatant 24 hours post infection. This concentration is almost 1000 times higher than the concentration achieved by the virus A/PR8/NS-1IL2StSt (Kittel et al., J. Virol. 79 (2005), 10672-10677) expressing IL-2 in the Stop-Start strategy and approximately 2-fold higher than when IL-2 was expressed from the complete NS1 deletion segment delNS1-IL-2-spl-mut3'-IgSP. Importantly, the IL-2 expression level was comparable after the $2^{nd}$ and $5^{th}$ passage of the virus, indicating that genetic stability correlated with stable transgene expression (Fig. 9b).

To confirm that cytokine expressed by NS-116-IL-2/A viruses possess the biological activity for the proliferation response of $CD3^+$ T lymphocytes after treatment with supernatants of infected Vero cells was analyzed. As shown in Fig. 10 proliferation of 27.5% $\pm$ 6.5% (n=6) $CD3^+CD4^+$ and 19% $\pm$ 8 % (n=6) of $CD3^+CD8^+$ was observed when cells were incubated with supernatant from the cells infected with IL-2 expressing virus, whereas just 6.5% $\pm$0.02% (n=2) and 2.7% $\pm$ 0.02% (n=2) of cells respectively, were proliferating after adding of NS-116-GFP/A derived supernatants.

Oncolytic effect of recombinant viruses

To test the oncolytic potential of the chimeric viruses NS-116-GFP/A or NS-116-GFP/A/IL-2 they were used for intratumoral injections in a syngeneic murine B16f1 model. Administration of both NS-116-/A viruses significantly inhibited tumor outgrowth, when compared with the control group. It should be noted, 25% of the animals treated with the NS-116-GFP/A/IL-2 virus never developed a tumor outgrowth after treatment, whereas mortality rate in the group treated with NS-116-GFP/A virus reached 100% (Fig. 11). Challenge of those long term survivors with $1x10^5$ B16F1 cells on day 70 did not lead to the development of tumors in any of the animals for an observation period of 70 further days.

Discussion

In the present experiments, the generation and analysis of a stable chimeric replicating influenza A virus vector is described, which expresses high levels of the different proteins such as GFP or IL-2 and which has the ability to grow well in cancer cells. This vector turned out to be the most stable replicating influenza A virus vector described so far. The generation of this vector was achieved by relying on evolutionary property of high mutability of influenza viruses leading to the ability to adapt. Most surprisingly, adaption of influenza A virus not only leads to enhanced growth but can also result in stabilization of the viral genome including stable maintenance of chimeric transgenes. This property renders the influenza A virus family suitable for construction of a clinical applicable vector with long insertions.

Adaptation of the parental chimeric influenza A virus vector NS-116-GFP/O to the NS-116-GFP/A vector was achieved by only 4 mutations scattered in PB2, HA and NS segments. This corresponds to the finding that only 5 mutations were responsible for the adaption of highly pathogenic avian influenza H5 viruses to replicate in ferrets. Thus, similar to adaption to new host virus, a few mutations seem to be sufficient for influenza virus to adapt to a insertion of a foreign gene, which spans at least half the size of a viral segment.

Most interestingly, stability was primarily dependent on a single mutation in the viral NEP protein. The (Q20R) mutation was found within the nuclear export signal (NES) sequence of the NEP, which spans for position 12 to 21. Correspondingly, mutations within this domain have been shown to attenuate a wild-type virus because of impaired nuclear export of viral RNPs. Vice versa, in this work the Q20R mutation allowed better growth and surprisingly adaption to a foreign insert. This correlates with a most recent finding that the mutations M16I in the NES is required for stable host adaptation of an avian virus to human cells. It was suggested that the interaction of the NEP with polymerase proteins might stabilize the latter in a host dependent manner. As mutations in the viral polymerase PB2 protein further add to high transgene expression and tumor adaptation of the here describe NS-116-GFP/A virus the data according to the present invention provide further evidence for an essential interaction of NEP and polymerase proteins for host adaptation and stability.

One requirement of a universally applicable influenza A virus vector is the possibility to exchange the external proteins. Therefore it was important that the exchange of the HA in NS-116/A viruses to the *A/New Caledonia/20/99* H1-subtype did not affect stability but only growth. It is well known that the exchange of the HAs can modulate the viral host restrictions. Also NS-116-GFP/A- and NS-116-IL-2/A H5 subtype viruses were constructed. Those isolates were not restricted in viral growth or in stability. This shows that the HA is readily exchangeable in the NS-116/A vector backbone.

Interestingly, it was found that the better growth of adapted NS-116-GFP/A virus and related mutants was associated with significantly less induction of IL-6, indicating that low IL-6 induction might be a surrogate parameter for virus adaption.

For generation of a stable virus viruses expressing a reporter gene were used, as this allowed to easily monitor transgene expression and thus genetic stability. However, the vector backbone maintained its stability, when a different transgene such as IL-2 has been used, suggesting some universal applicability for chimeric influenza A virus vectors. This is supported by the fact that also other cytokines or foreign antigens could be stably expressed in this viral vector. Initially, Vero cells were used to select for the highly expressing chimeric virus. The vector design according to the present invention will complement previous designed chimeric influenza A virus vectors. Comparison of different vectors will provide a better understanding of the genetic elements of each vector and will allow the choice of the appropriate vector for each indication. The NS-116/A vector expressed the IL-2 transgene at similar high levels as the previously described replication defective delNS1 vector, for which the cytokine secretion was achieved through a fusion of N-terminal 13 amino acids of NS1 with a modified IgK signal peptide.

As the length of NS1-116 prohibited a direct fusion with a signal peptide in order to achieve IL-2 secretion a 2A autoproteolytic cleavage site was inserted. Secretion of functional IL-2 into the supernatant was clearly demonstrated.

Kittel et al. (J. Virol. 79 (2005), 10672-10677) have designed an influenza vector in which IL-2 is expressed via a Stop-Start motif downstream of a partially deleted NS protein. This virus appeared to be genetically stable but expressed only low levels of the transgene in a range of 250-300pg/ml, indicating that the Stop-Start motif is far less potent than the 2A cleavage site in the influenza A virus background.

Different chimeric influenza A viruses, which have been generated might be used for different purposes in the future. The high transgene expression levels in combination with a replicating phenotype renders the NS-116/A vector most appropriate for oncolytic purposes but also as an antigenic vaccine vector, as it is less affected by the host's IFN response. In contrast, the replication-defective delNS1 vector might be specifically appropriate for a vaccine vector in compromised people such as the elderly or in infants as this vector depicts the highest safety profile. The Stop-Start motif vector might be useful to express proteins, which would be too toxic at higher concentrations.

Most recently, Manicassamy et al. (PNAS 107 (2010), 11531-11536) generated a GFP-expressing influenza virus containing full length NS1 to track the virus in the host. GFP was fused to the ORF of the NEP protein separated by the 2A cleavage site. *In vivo* experiments showed that 30% of virus population in lung homogenates were carrying deletions in the chimeric GFP segment on the day 6 postinfection, indicating, that the 2A autocatalytic cleavage site by itself is not providing stability to the transgene.

The vector according to the present invention was applied to a very aggressive syngeneic murine tumor model. Previously the replication deficient delNS1 vector expressing IL-2 was tested in this model. Whereas the empty replication deficient delNS1 virus was not associated with any benefit, the delNS1-IL-2 expressing vector delayed survival by approximately 1 week. The over-attenuation of the delNS1 vector used might prevent an efficient therapeutic effect. In contrast, intratumoral injection of both, the progenitor NS-116-GFP/A virus and modified NS-116-IL2/A virus remarkable slowed down tumor growth in comparison with the control, indicating that the sole use of a replicating B16f1-adapted virus has a therapeutic impact. This also supports the observation that replicating influenza A viruses have better therapeutic effect than non-replicating less IFN sensitive vectors. The further benefit of the transgene is documented by the fact, that 25% of the animals in the group with NS-116-IL2/A treatment showed complete regression of the tumor and remained tumor free even after a second implantation of cancer cells (Fig. 11). Thus, efficient oncolysis with influenza A viruses should employ a tumor-adapted conditionally replicating virus expressing high levels of a therapeutic transgene. IL-2 was used as a transgene, as it has been shown to be superior to other cytokines when inserted into the genome of other oncolytic RNA viruses, such as NDV. However, that fact that this cytokine is associated with the generation of regulatory T cells (Treg) suggests that other pro-inflammatory therapeutic cytokines might be even more beneficial and therefore should be tested.

Most importantly, the present invention shows that despite the high mutation rate of the influenza A virus, stable transgenic viruses can be generated by virtue of self-optimization of the virus. The genetic stability is maintained in vivo allowing therapeutic application of such chimeric vector constructs.

2. Modification of HA cleavage site influenza A virus for oncolytic tumor therapy

In this example, the exchange of the trypsin cleavage sites in the Flu A hemagglutinin by an elastase cleavage site is introduced as an additional preferred embodiment into the vector according to the present invention.

The principle of this aspect is depicted in Fig. 12: A second attenuation marker is inserted in the NS-116-GFP/A virus. This is an altered protease cleavage site on the viral entry protein, the hemagglutinin. Exchange of the trypsin cleavage site, present in wild type virus to an elastase cleavage site usually leads to attenuation of the virus (Stech et al., Nature Medicine 11 (2005), 683 - 689. This attenuation allows the use of an H5 subtype, as the HA is linked to the attenuation marker.

The tumor infiltrating neutrophilic granulocytes, which produce elastase will activate the virus in the tumor. It was therefore shown that AE viruses can be activated by neutrophilic elastase and by SN of neutrophilic granulocytes as seen by fluorescence of the transgene GFP (Fig. 13a and b).

Plaque assay analysis showed that viral growth of AE viruses under conditions, where elastase was supplied only 50 times less than growth of the wild type AT virus, when the later was activated by trypsin. This shows that elastase should support efficient replication of an elastase dependent virus(Fig. 14).

A murine model showed that virus with changed cleavage site (AE) still effectively reduces growth of pancreatic cancer cells (Fig. 15). The tumor reduction was significantly better than achieved with a conventional AE virus.

Table 1

Nucleotide and amino acid changes of NS-116-GFP/A virus in comparison with NS-116-GFP/O virus.

| Gene | Virus | Nucleotide position | Nucleotide | Amino acid position | Amino acid |
|---|---|---|---|---|---|
| PB2 | NS-116-GFP/O | 1632 | G | 535 | M |
| | NS-116-GFP/A | | A | | I |
| HA | NS-116-GFP/O | 1467 | G | 479 | G |
| | NS-116-GFP/A | | A | | R |
| NS1-116-GFP | NS-116-GFP/O | 1128 | T | 368 | Y |
| | NS-116-GFP/A | | C | | H |
| NEP | NS-116-GFP/O | 1335 | A | 20 | Q |
| | NS-116-GFP/A | | G | | R |

Table 2

Maximal viral titers and stability of "adapted-change-reversion"-viruses.

| Virus name | Segment/aa position | | | | Max viral titer, $log_{10}$ $TCID_{50}$/ml | | Stability: %GFP+ plaques from total amount of plaques after 5 passages | |
|---|---|---|---|---|---|---|---|---|
| | NEP **20** | NS1-GFP **368** | PB2 **535** | HA **479** | B16f1 | Vero | B16f1 | Vero |
| NS-116-GFP/O | Q | Y | M | G | 4.5±0.1* | 7.3±0.2 | 42,7±9.0* | 16,7±2.4* |
| NS-116-GFP/A | R | H | I | R | 6.9±0.1 | 8.0±0.2 | 96,5±1.7 | 97.6±1.4 |
| NS-116-GFP/A/NS 368 | R | Y | I | R | 3.5±0.3* | 6.1±0.3* | 83.1±5.7 | 95,3.4±3.2 |

(continued)

| Maximal viral titers and stability of "adapted-change-reversion"-viruses. | | | | | | | | |
|---|---|---|---|---|---|---|---|---|
| Virus name | Segment/aa position | | | | Max viral titer, $log_{10}$ $TCID_{50}$/ml | | Stability: %GFP+ plaques from total amount of plaques after 5 passages | |
| | NEP **20** | NS1-GFP **368** | PB2 **535** | HA **479** | B16f1 | Vero | B16f1 | Vero |
| NS-116-GFP/A/NEP20 | Q | H | I | R | 4.4±0.4* | 6.2±0.3* | 53±11.5* | 14.7±4.8* |
| NS-116-GFP/A/PB 2 535 | R | H | M | R | 6.0±0.4 | 7.3±0.3 | 91±4.8 | 79.3±13.0 |
| NS-116/A/PB 2 535 NS368 | R | Y | M | R | 7.9±0.5 | 6.9±0.4 | 90.3±3.5 | 93.9±2.4 |
| NS-116/A/HA | R | H | I | HA segment from NC° | 4.7±0.2* | 5.3±0.3* | 94.6±2.3 | 98.0±0.8 |

Data are presented as means ± SEM (n=3 for titer values, n=6 for stability values)
•P<0.05, comparing with NS-116-GFP/A
• ° the whole segment was exchanged

Table 3

Absolute level of IL-6 produced by infected B16f1 cells, ng/ml .

| Virus name \ Time post infection | 12 hpi | 24 hpi | 48 hpi |
|---|---|---|---|
| mock | 0 | 0 | 0 |
| NS-116-GFP/O | 0 | 331.2 | 658.9 |
| NS-116-GFP/A | 0 | 110.4 | 114.8 |
| NS-116-GFP/A/NS368 | 0 | 845.9 | 935.0 |
| NS-116-GFP/A/NEP20 | 322.9 | 1313.9 | 1367.6 |
| NS-116-GFP/A/PB2 535 | 0 | 30.5 | 401.9 |
| NS-116/A/PB2 535 NS368 | 0 | 118.9 | 288.1 |

Table 4

Sequence of influenza virus A/PR/8/34 (H1N1)

NA:
AGCGAAAGCAGGAGTTTAAAATGAATCCAAATCAGAAAATAATAACCATTGGATCAATCTGTCTGGTAGTCGGACTAATTAGCCTAATATTGCAAAT
AGGGAATATAATCTCAATATGGATTAGCCATTCAATTCAAACTGGAAGTCAAAACCATACTGGAATATGCAACCAAAACATCATTACCTATAAAAAT
AGCACCTGGGTAAAGGACACAACTTCAGTGATATTAACCGGCAATTCATCTCTTTGTCCCATCCGTGGGTGGGCTATATACAGCAAAGACAATGGCA
TAAGAATTGGTTCCAAAGGAGACGTTTTTGTCATAAGAGAGCCCTTTATTTCATGTTCTCACTTGGAATGCAGGACCTTTTTTCTGACCCAAGGTGC
CTTACTGAATGACAAGCATTCAAGTGGGACTGTTAAGGACAGAAGCCCTTATAGGGCCTTAATGAGCTGCCCTGTCGGTGAAGCTCCGTCCCCGTAC
AATTCAAGATTTGAATCGGTTGCTTGGTCAGCAAGTGCATGTCATGATGGCATGGGCTGGCTAACAATCGGAATTTCAGGTCCAGATAATGGAGCAG
TGGCTGTATTAAAATACAACGGCATAATAACTGAAACCATAAAAAGTTGGAGGAAGAAAATATTGAGGACACAAGAGTCTGAATGTGCCTGTGTAAA
TGGTTCATGTTTTACTATAATGACTGATGGCCCGAGTGATGGGCTGGCCTCATACAAAATTTTCAAGATCGAAAAGGGGAAGGTTACTAAATCAATA
GAGTTGAATGCACCTAATTCTCACTATGAGGAATGTTCCTGTTACCCTGATACCGGCAAAGTGATGTGTGTGTGCAGAGACAATTGGCATGGTTCGA
ACCGGCCATGGGTGTCTTTCGATCAAAACCTGGATTATCAAATAGGATACATCTGCAGTGGGGTTTTCGGTGACAACCCGCGTCCCGAAGATGGAAC
AGGCAGCTGTGGTCCAGTGTATGTTGATGGAGCAAACGGAGTAAAGGGATTTTCATATAGGTATGGTAATGGTGTTTGGATAGGAAGGACCAAAAGT
CACAGTTCCAGACATGGGTTTGAGATGATTTGGGATCCTAATGGATGGACAGAGACTGATAGTAAGTTCTCTGTGAGGCAAGATGTTGTGGCAATGA
CTGATTGGTCAGGGTATAGCGGAAGTTTCGTTCAACATCCTGAGCTGACAGGGCTAGACTGTATGAGGCCGTGCTTCTGGGTTGAATTAATCAGGGG
ACGACCTAAAGAAAAACAATCTGGACTAGTGCGAGCAGCATTTCTTTTTGTGGCGTGAATAGTGATACTGTAGATTGGTCTTGGCCAGACGGTGCT
GAGTTGCCATTCAGCATTGACAAGTAGTCTGTTCAAAAAACTCCTTGTTTCTACT
NP:
AGCAAAAGCAGGGTAGATAATCACTCACTGAGTGACATCAAAATCATGGCGTCCCAAGGCACCAAACGGTCTTACGAACAGATGGAGACTGATGGAG
AACGCCAGAATGCCACTGAAATCAGAGCATCCGTCGGAAAAATGATTGGTGGAATTGGACGATTCTACATCCAAATGTGCACCGAACTCAAACTCAG
TGATTATGAGGGACGGTTGATCCAAAACAGCTTAACAATAGAGAGAATGGTGCTCTCTGCTTTTGACGAAAGGAGAAATAAATACCTGGAAGAACAT
CCCAGTGCGGGGAAAGATCCTAAGAAAACTGGAGGACCTATATACAGGAGAGTAAACGGAAAGTGGATGAGAGAACTCATCCTTTATGACAAAGAAG
AAATAAGGCGAATCTGGCGCCAAGCTAATAATGGTGACGATGCAACGGCTGGTCTGACTCACATGATGATCTGGCATTCCAATTTGAATGATGCAAC
TTATCAGAGGACAAGAGCTCTTGTTCGCACCGGAATGGATCCCAGGATGTGCTCTCTGATGCAAGGTTCAACTCTCCCTAGGAGGTCTGGAGCCGCA
GGTGCTGCAGTCAAAGGAGTTGGAACAATGGTGATGGAATTGGTCAGGATGATCAAACGTGGGATCAATGATCGGAACTTCTGGAGGGGTGAGAATG
GACGAAAAACAAGAATTGCTTATGAAAGAATGTGCAACATTCTCAAAGGGAAATTTCAAACTGCTGCACAAAAAGCAATGATGGATCAAGTGAGAGA
GAGCCGGAACCCAGGGAATGCTGAGTTCGAAGATCTCACTTTTCTAGCACGGTCTGCACTTATATTGAGAGGGTCGGTTGCTCACAAGTCCTGCCTG
CCTGCCTGTGTGTATGGACCTGCCGTAGCCAATGGGTACGACTTTGAAAGAGAGGGATACTCTCTAGTCGGAATAGACCCTTTCAGACTGCTTCAAA
ACAGCCAAGTGTACAGCCTAATCAGACCAAATGAGAATCCAGCACACAAGAGTCAACTGGTGTGGATGGCATGCCATTCTGCCGCATTTGAAGATCT
AAGAGTATTAAGCTTCATCAAAGGGACGAAGGTGCTCCCAAGAGGGAAGCTTTCCACTAGAGGAGTTCAAATTGCTTCCAATGAAAATATGGAGACT
ATGGAATCAAGTACACTTGAACTGAGAAGCAGGTACTGGGCCATAAGGACCAGAAGTGGAGGAAACACCAATCAACAGAGGGCATCTGCGGGCCAAA
TCAGCATACAACCTACGTTCTCAGTACAGAGAAATCTCCCTTTTGACAGAACAACCATTATGGCAGCATTCAATGGGAATACAGAGGGGAGAACATC
TGACATGAGGACCGAAATCATAAGGATGATGGAAAGTGCAAGACCAGAAGATGTGTCTTTCCAGGGGCGGGGAGTCTTCGAGCTCTCGGACGAAAAG
GCAGCGAGCCCGATCGTGCCTTCCTTTGACATGAGTAATGAAGGATCTTATTTCTTCGGAGACAATGCAGAGGAGTACGACAATTAAAGAAAAATAC
CCTTGTTTCTACT
NS116:
AGCAAAAGCAGGGTGACAAAGACATAATGGATCCAAACACTGTGTCAAGCTTTCAGGTAGATTGCTTTCTTTGGCATGTCCGCAAACGAGTTGCAGA
CCAAGAACTAGGTGATGCCCCATTCCTTGATCGGCTTCGCCGAGATCAGAAATCCCTAAGAGGAAGGGGCAGCACCCTCGGTCTGGACATCGAGACA
GCCACACGTGCTGGAAAGCAGATAGTGGAGCGGATTCTGAAAGAAGAATCCGATGAGGCACTTAAAATGACCATGGCCTCTGTACCTGCGTCGCGTT
ACCTAACTGACATGACTCTTGAGGAAATGTCAAGGGACTGGTCCATGTCTTTGTATCAGAATGGACCAGGCGATCATGGATTAATGGCTAGCAAAGG
AGAAGAACTCTTCACTGGAGTTGTCCCAATTCTTGTTGAATTAGATGGTGATGTTAACGGCCACAAATTCTCTGTCAGTGGAGAGGGTGAAGGTGAT
GCAACATACGGAAAACTTACCCTGAAGTTCATCTGCACTACTGGCAAACTGCCTGTTCCATGGCCAACACTAGTCACTACTCTGTGCTATGGTGTTC
AATGCTTTTCAAGATACCCGGATCATATGAAACGGCATGACTTTTTCAAGAGTGCCATGCCCGAAGGTTATGTACAGGAAAGGACCATCTTCTTCAA
AGATGACGGCAACTACAAGACACGTGCTGAAGTCAAGTTTGAAGGTGATACCCTTGTTAATAGAATCGAGTTAAAAGGTATTGACTTCAAGGAAGAT
GGCAACATTCTGGGACACAAATTGGAATACAACTATAACTCACACAATGTATACATCATGGCAGACAAACAAAAGAATGGAATCAAAGTGAACTTCA
AGACCCGCCACAACATTGAAGATGGAAGCGTTCAACTAGCAGACCATTATCAACAAAATACTCCAATTGGCGATGGCCCTGTCCTTTTACCAGACAA
CCATTACCTGTCCACACAATCTGCCCTTTCGAAAGATCCCAACGAAAAGAGAGACCACATGGTCCTTCTTGAGTTTGTAACAGCTGCTGGGATTACA
CATGGCATGGATGAACTGTACAACATCGATGGAGGCGGAGGTGGAGGGCCCGGTTACCGGCACCGGATCCAGATATCTGGGCGGCCGCTCAGCAAGC
TTCGCGAATTCTGATAAGAACATCATACTGAAAGCGAACTTCAGTGTGATTTTTGACCGGCTGGAGACTCTAATATTGCTAAGGGCTTTCACCGAAG
AGGGAGCAATTGTTGGCGAAATTTCACCATTGCCTTCTCTTCCAGGACATACTGCTGAGGATGTCAAAAATGCGGTTGGAGTCCTCATCGGGGGACT
TGAAAGGAATGATAACACAGTTCGAGTCTCTGAAACTCTACAGAGATTCGCTTGGAGAAGCAGTAATGAGAATGGGAGACCTCCACTCACTCCAAAA
CAGAAACGAGAAATGGCGGGAACAATTAGGTCAGAAGTTTGAAGAAATAAGATGGTTGATTGAAGAAGTGAGACACAAACTGAAGATAACAGAGAAT
AGTTTTGAGCAAATAACATTTATGCAAGCCTTACATCTATTGCTTGAAGTGGAGCAAGAGATAAGAACTTTCTCGTTTCAGCTTATTTAGTAATAAA
AAACACCCTTGTTTCTACT
PA:
AGCGAAAGCAGGTACTGATCCAAAATGGAAGATTTTGTGCGACAATGCTTCAATCCGATGATTGTCGAGCTTGCGGAAAAAACAATGAAAGAGTATG
GGGAGAACCTGAAAATCGAAACAAACAAATTTGCAGCAATATGCACTCACTTGGAAGTATGCTTCATGTATTCAGATTTTCACTTCATCAATGAGCA

AGGCGAGTCAATAATCGTAGAACTTGGTGATCCAAATGCACTTTTGAAGCACAGATTTGAAATAATCGAGGGAAGAGATCGCACAATGGCCTGGACA
GTAGTAAACAGTATTTGCAACACTACAGGGGCTGAGAAACCAAAGTTCCTACCAGATTTGTATGATTACAAGGAGAATAGATTCATCGAAATTGGAG
TAACAAGGAGAGAAGTTCACATATACTATCTGGAAAAGGCCAATAAAATTAAATCTGAGAAAACACACATCCACATTTTCTCGTTCACTGGGGAAGA
AATGGCCACAAAGGCAGACTACACTCTCGATGAAGAAAGCAGGGCTAGGATCAAAACCAGACTATTCACCATAAGACAAGAAATGGCCAGCAGAGGC
CTCTGGGATTCCTTTCGTCAGTCCGAGAGAGGAGAAGAGACAATTGAAGAAAGGTTTGAAATCACAGGAACAATGCGCAAGCTTGCCGACCAAAGTC
TCCCGCCGAACTTCTCCAGCCTTGAAAATTTTAGAGCCTATGTGGATGGATTCGAACCGAACGGCTACATTGAGGGCAAGCTGTCTCAAATGTCCAA
AGAAGTAAATGCTAGAATTGAACCTTTTTTGAAAACAACACCACGACCACTTAGACTTCCGAATGGGCCTCTCTGTTCTCAGCGGTCCAAATTCCTG
CTGATGGATGCCTTAAAATTAAGCATTGAGGACCCAAGTCATGAAGGAGAGGGAATACCGCTATATGATGCAATCAAATGCATGAGAACATTCTTTG
GATGGAAGGAACCCAATGTTGTTAAACCACACGAAAAGGGAATAAATCCAAATTATCTTCTGTCATGGAAGCAGGTACTGGCAGAACTGCAGGACAT
TGAGAATGAGGAGAAAATTCCAAAGACTAAAATTATGAAGAAAACAAGTCAGCTAAAGTGGGCACTTGGTGAGAACATGGCACCAGAAAAGGTAGAC
TTTGACGACTGTAAAGATGTAGGTGATTTGAAGCAATATGATAGTGATGAACCAGAATTGAGGTCGCTTGCAAGTTGGATTCAGAATGAGTTTAACA
AGGCATGCGAACTGACAGATTCAAGCTGGATAGAGCTCGATGAGATTGGAGAAGATGTGGCTCCAATTGAACACATTGCAAGCATGAGAAGGAATTA
TTTCACATCAGAGGTGTCTCACTGCAGAGCCACAGAATACATAATGAAGGGGGTGTACATCAATACTGCCTTGCTTAATGCATCTTGTGCAGCAATG
GATGATTTCCAATTAATTCCAATAATAAGCAAGTGTAGAACTAAGGAGGGAAGGCGAAAGACCAACTTGTATGGTTTCATCATAAAAGGAAGATCCC
ACTTAAGGAATGACACCGACGTGGTAAACTTTGTGAGCATGGAGTTTTCTCTCACTGACCCAAGACTTGAACCACATAAATGGGAGAAGTACTGTGT
TCTTCACATACCACATATCCTTATAACAACTCCCATACCCCAGCTTTCAAGGCCCATCTTCTTCTATCTCACAACAAATCCAACCTCAAAAATTAAA
ATGAAATGGGGAATGGAGATGAGGCGTTGCCTCCTCCAGTCACTTCAACAAATTGAGAGTATGATTGAAGCTGAGTCCTCTGTCAAAGAGAAAGACA
TGACCAAAGAGTTCTTTGAGAACAAATCAGAAACATGGCCCATTGGAGAGTCCCCCAAAGGAGTGGAGGAAAGTTCCATTGGGAAGGTCTGCAGGAC
TTTATTAGCAAAGTCGGTATTCAACAGCTTGTATGCATCTCCACAACTAGAAGGATTTTCAGCTGAATCAAGAAAACTGCTTCTTATCGTTCAGGCT
CTTAGGGACAACCTTGAACCTGGGACCTTTAATCTTGGGGGGCTATATGAAGCAATTGAGGAGTGCCTGATTAATGATCCCTGGGTTTTGCTTAATG
CTTCTTGGTTCAACTCCTTCCTTACACATGCATTGAGTTAGTTGTGGCAGTGCTACTATTTGCTATCCATACTGTCCAAAAAAGTACCTTGTTTCTA
CT

PB1:

AGCGAAAGCAGGCAAACCATTTGAATGGATGTCAATCCGACCTTACTTTTCTTAAAAGTGCCAGCACAAAATGCTATAAGCACAACTTTCCCTTATA
CTGGAGACCCTCCTTACAGCCATGGGACAGGAACAGGATACACCATGGATACTGTCAACAGGACACATCAGTACTCAGAAAAGGGAAGATGGACAAC
AAACACCGAAACTGGAGCACCGCAACTCAACCCGATTGATGGGCCACTGCCAGAAGACAATGAACCAAGTGGTTATGCCCAAACAGATTGTGTATTG
GAGGCGATGGCTTTCCTTGAGGAATCCCATCCTGGTATTTTTGAAAACTCGTGTATTGAAACGATGGAGGTTGTTCAGCAAACACGAGTAGACAAGC
TGACACAAGGCCGACAGACCTATGACTGGACTCTAAATAGAAACCAACCTGCTGCAACAGCATTGGCCAACACAATAGAAGTGTTCAGATCAAATGG
CCTCACGGCCAATGAGTCTGGAAGGCTCATAGACTTCCTTAAGGATGTAATGGAGTCAATGAACAAAGAAGAAATGGGGATCACAACTCATTTTCAG
AGAAAGAGACGGGTGAGAGACAATATGACTAAGAAAATGATAACACAGAGAACAATGGGTAAAAAGAAGCAGAGATTGAACAAAAGGAGTTATCTAA
TTAGAGCATTGACCCTGAACACAATGACCAAAGATGCTGAGAGAGGGAAGCTAAAACGGAGAGCAATTGCAACCCCAGGGATGCAAATAAGGGGGTT
TGTATACTTTGTTGAGACACTGGCAAGGAGTATATGTGAGAAACTTGAACAATCAGGGTTGCCAGTTGGAGGCAATGAGAAGAAAGCAAAGTTGGCA
AATCTTCTAACCAACATGATCACCAATTCTCAGCACACCCAACTTTCTTTCACCATCACTCCACATAACACCAAATCCAACCAAAATCACAATCCTC
GGATGTTTTTGGCCATGATCACATATATGACCAGAAATCAGCCCGAATGGTTCAGAAATGTTCTAAGTATTGCTCCAATAATGTTCTCAAACAAAAT
GGCGAGACTGGGAAAAGGGTATATGTTTGAGAGCAAGAGTATGAAACTTAGAACTCAAATACCTGCAGAAATGCTAGCAAGCATCGATTTGAAATAT
TTCAATGATTCAACAAGAAAGAAGATTGAAAAAATCCGACCGCTCTTAATAGGGGGGACTGCATCATTGAGCCCTGGAATGATGATGGGCATGTTCA
ATATGTTAAGCACTGTATTAGGCGTCTCCATCCTGAATCTTGGACAAAAGAGATACACCAAGACTACTTACTGGTGGGATGGTCTTCAATCCTCTGA
CGATTTTGCTCTGATTGTGAATGCACCCAATCATGAAGGGATTCAAGCCGGAGTCGACAGGTTTTATCGAACCTGTAAGCTACTTGGAATCAATATG
AGCAAGAAAAAGTCTTACATAAACAGAACAGGTACATTTGAATTCACAAGTTTTTTCTATCGTTATGGGTTTGTTGCCAATTTCAGCATGGAGCTTC
CCAGTTTTGGGGTGTCTGGGATCAACGAGTCAGCGGACATGGGTATTGGAGTTACTGTCATCAAAAACAATATGATAAACAATGATCTTGGTCCAGC
AACAGCTCAAATGGCCCTTCAGTTGTTCATCAAAGATTACAGGTACACGTACCGATGCCATAGAGGTGACACACAAATACAAACCCGAAGATCATTT
GAAATAAAGAAACTGTGGGAGCAAACCCGTTCCAAAGCTGGACTGCTGGTCTCCGACGGAGGCCCAAATTTATACAACATTAGAAATCTCCACATTC
CTGAAGTCTGCCTAAAATGGGAATTGATGGATGAGGATTACCAGGGGCGTTTATGCAACCCACTGAACCCATTTGTCAGCCATAAAGAAATTGAATC
AATGAACAATGCAGTGATGATGCCAGCACATGGTCCAGCCAAAAACATGGAGTATGATGCTGTTGCAACAACACACTCCTGGATCCCCAAAAGAAAT
CGATCCATCTTGAATACAAGTCAAAGAGGAGTACTTGAGGATGAACAAATGTACCAAAGGTGCTGCAATTTATTTGAAAAATTCTTCCCCAGCAGTT
CATACAGAAGACCAGTCGGGATATCCAATATGGTGGAGGCTATGGTTTCCAGAGCCCGAATTGATGCACGGATTGATTTCGAATCTGGAAGGATAAA
GAAAGAAGAGTTCACTGAGATCATGAAGATCTGTTCCACCATTGAAGAGCTCAGACGGCAAAAATAGTGAATTTAGCTTGTCCTTCATGAAAAAATG
CCTTGTTTCTACT

PB2:

AGCGAAAGCAGGTCAATTATATTCAATATGGAAAGAATAAAAGAACTAAGAAATCTAATGTCGCAGTCTCGCACCCGCGAGATACTCACAAAAACCA
CCGTGGACCATATGGCCATAATCAAGAAGTACACATCAGGAAGACAGGAGAAGAACCCAGCACTTAGGATGAAATGGATGATGGCAATGAAATATCC
AATTACAGCAGACAAGAGGATAACGGAAATGATTCCTGAGAGAAATGAGCAAGGACAAACTTTATGGAGTAAAATGAATGATGCCGGATCAGACCGA
GTGATGGTATCACCTCTGGCTGTGACATGGTGGAATAGGAATGGACCAATAACAAATACAGTTCATTATCCAAAAATCTACAAAACTTATTTTGAAA
GAGTCGAAAGGCTAAAGCATGGAACCTTTGGCCCTGTCCATTTTAGAAACCAAGTCAAAATACGTCGGAGAGTTGACATAAATCCTGGTCATGCAGA
TCTCAGTGCCAAGGAGGCACAGGATGTAATCATGGAAGTTGTTTTCCCTAACGAAGTGGGAGCCAGGATACTAACATCGGAATCGCAACTAACGATA
ACCAAAGAGAAGAAAGAAGAACTCCAGGATTGCAAAATTTCTCCTTTGATGGTTGCATACATGTTGGAGAGAGAACTGGTCCGCAAAACGAGATTCC
TCCCAGTGGCTGGTGGAACAAGCAGTGTGTACATTGAAGTGTTGCATTTGACTCAAGGAACATGCTGGGAACAGATGTATACTCCAGGAGGGGAAGT
GAGGAATGATGATGTTGATCAAAGCCTGATTATTGCTGCTAGGAACATAGTGAGAAGAGCTGCAGTATCAGCAGATCCACTAGCATCTTTATTGGAG
ATGTGCCACAGCACACAGATTGGTGGAATTAGGATGGTAGACATCCTTAGGCAGAACCCAACAGAAGAGCAAGCCGTGGATATATGCAAGGCTGCAA
TGGGACTGAGAATTAGCTCATCCTTCAGTTTTGGTGGATTCACATTTAAGAGAACAAGCGGATCATCAGTCAAGAGAGAGGAAGAGGTGCTTACGGG
CAATCTTCAAACATTGAAGATAAGAGTGCATGAGGGATATGAAGAGTTCACAATGGTTGGGAGAAGAGCAACAGCCATACTCAGAAAAGCAACCAGG
AGATTGATTCAGCTGATAGTGAGTGGGAGAGACGAACAGTCGATTGCCGAAGCAATAATTGTGGCCATGGTATTTTCACAAGAGGATTGTATGATAA
AAGCAGTCAGAGGTGATCTGAATTTCGTCAATAGGGCGAATCAGCGATTGAATCCTATGCATCAACTTTTAAGACATTTTCAGAAGGATGCGAAAGT
GCTTTTTCAAAATTGGGGAGTTGAACCTATCGACAATGTGATGGGAATGATTGGGATATTGCCCGACATGACTCCAAGCATCGAGATGTCAATGAGA
GGAGTGAGAATCAGCAAAATGGGTGTAGATGAGTACTCCAGCACGGAGAGGGTAGTGGTGAGCATTGACCGTTTTTTGAGAATCCGGGACCAACGAG
GAAATCTACTACTGTCTCCCGAGGAGGTCAGTGAAACACAGGGAACAGAGAAACTGACAATAACTTACTCATCCTCAATAATGTGGGAGATTAATGG
TCCTGAATCAGTGTTGGTCAATACCTATCAATGGATCATCAGAAACTGGGAAACTATTAAAATTCAGTGGTCCCAGAACCCTACAATGCTATACAAT
AAAATGGAATTTGAACCATTTCAGTCTTTAGTACCTAAGGCCATTAGAGGCCAATACAGTGGGTTTGTAAGAACTCTGTTCCAACAAATGAGGGATG
TGCTTGGGACATTTGATACCGCACAGATAATAAAACTTCTTCCCTTCGCAGCCGCTCCACCAAAGCAAAGTAGAATGCAGTTCTCCTCATTTACTGT
GAATGTGAGGGGATCAGGAATGAGAATACTTGTAAGGGGCAATTCTCCTGTATTCAACTATAACAAGGCCACGAAGAGACTCACAGTTCTCGGAAAG
GATGCTGGCACTTTAACTGAAGACCCAGATGAAGGCACAGCTGGAGTGGAGTCCGCTGTTCTGAGGGGATTCCTCATTCTGGGCAAAGAAGACAAGA
GATATGGGCCAGCACTAAGCATCAATGAACTGAGCAACCTTGCGAAAGGAGAGAAGGCTAATGTGCTAATTGGGCAAGGAGACGTGGTGTTGGTAAT
GAAACGGAAACGGGACTCTAGCATACTTACTGACAGCCAGACAGCGACCAAAAGAATTCGGATGGCCATCAATTAGTGTCGAATAGTTTAAAAACGA
CCTTGTTTCTACT

HA:

AGCAAAAGCAGGGGAAAATAAAAACAACCAAAATGAAGGCAAACCTACTGGTCCTGTTATGTGCACTTGCAGCTGCAGATGCAGACACAATATGTAT
AGGCTACCATGCGAACAATTCAACCGACACTGTTGACACAGTACTCGAGAAGAATGTGACAGTGACACACTCTGTTAACCTGCTCGAAGACAGCCAC

AACGGAAAACTATGTAGATTAAAAGGAATAGCCCCACTACAATTGGGGAAATGTAACATCGCCGGATGGCTCTTGGGAAACCCAGAATGCGACCCAC
TGCTTCCAGTGAGATCATGGTCCTACATTGTAGAAACACCAAACTCTGAGAATGGAATATGTTATCCAGGAGATTTCATCGACTATGAGGAGCTGAG
GGAGCAATTGAGCTCAGTGTCATCATTCGAAAGATTCGAAATATTTCCCAAAGAAAGCTCATGGCCCAACCACAACACAAACGGAGTAACGGCAGCA
TGCTCCCATGAGGGGAAAAGCAGTTTTTACAGAAATTTGCTATGGCTGACGGAGAAGGAGGGCTCATACCCAAAGCTGAAAAATTCTTATGTGAACA
AAAAAGGGAAAGAAGTCCTTGTACTGTGGGGTATTCATCACCCGCCTAACAGTAAGGAACAACAGAATCTCTATCAGAATGAAAATGCTTATGTCTC
TGTAGTGACTTCAAATTATAACAGGAGATTTACCCCGGAAATAGCAGAAAGACCCAAAGTAAGAGATCAAGCTGGGAGGATGAACTATTACTGGACC
TTGCTAAAACCCGGAGACACAATAATATTTGAGGCAAATGGAAATCTAATAGCACCAATGTATGCTTTCGCACTGAGTAGAGACTTTGGGTTCGGCA
TCATCACCTCAAACGCATCAATACATGAGTGTAACACGAAGTGTCAAACACCCCTGGGAGCTATAAACAGCAGTCTCCCTTACCAGAATATACACCC
AGTCACAATAGGAGAGTGCCCAAAATACGTCAGGAGTGCCAAATTGAGGATGGTTACAGGACTAAGGAACATTCCGTCCATTCAATCCAGAGGTCTA
TTTGGAGCCTTTGCCGGTTTTATTGAAGGGGGATGGACTGGAATGATAGATGGATGGTATGGTTATCATCATCAGAATGAACAGGGATCAGGCTATG
CAGCGGATCAAAAAAGCACACAAAATGCCATTAACGGAATTACAAACAAGGTGAACACTGTTATCGAGAAAATGAACATTCAATTCACAGCTGTGGG
TAAAGAATTCAACAAATTAGAAAAAAGGATGGAAAATTTAAATAAAAAAGTTGATGATGGATTTCTGGACATTTGGACATATAATGCAGAATTGTTA
GTTCTACTGGAAAATGAAAGGACTCTGGATTTCCATGACTCAAATGTGAAGAATCTGTATGAGAAAGTAAAAAGCCAATTAAAGAATAATGCCAAAG
AAATCGGAAATAGATGTTTTGAGTTCTACCACAAGTGTGACAATGAATGCATGGAAAGTGTAAGAAATGGGACTTATGATTATCCCAAATATTCAGA
AGAGTCAAAGTTGAACAGGGAAAAGGTAGATGGAGTGAAATTGGAATCAATGGGGATCTATCAGATTCTGGCGATCTACTCAACTGTCGCCAGTTCA
CTGGTGCTTTTGGTCTCCCTGGGGGCAATCAGTTTCTGGATGTGTTCTAATGGATCTTTGCAGTGCAGAATATGCATCTGAGATTAGAATTTCAGAG
ATATGAGGAAAAACACCCTTGTTTCTACT

M:

AGCGAAAGCAGGTAGATATTGAAAGATGAGTCTTCTAACCGAGGTCGAAACGTACGTACTCTCTATCATCCCGTCAGGCCCCCTCAAAGCCGAGATC
GCACAGAGACTTGAAGATGTCTTTGCAGGGAAGAACACCGATCTTGAGGTTCTCATGGAATGGCTAAAGACAAGACCAATCCTGTCACCTCTGACTA
AGGGGATTTTAGGATTTGTGTTCACGCTCACCGTGCCCAGTGAGCGAGGACTGCAGCGTARACGCTTTGTCCAAAATGCCCTTAATGGGAACGGGGA
TCCAAATAACATGGACAAAGCAGTTAAACTGTATAGGAAGCTCAAGAGGGAGATAACATTCCATGGGGCCAAAGAAATCTCACTCAGTTATTCTGCT
GGTGCACTTGCCAGTTGTATGGGCCTCATATACAACAGGATGGGGGCTGTGACCACTGAAGTGGCATTTGGCCTGGTATGTGCAACCTGTGAACAGA
TTGCTGACTCCCAGCATCGGTCTCATAGGCAAATGGTTACAACAACCAATCCACTAATCAGACATGAGAACAGAATGGTTTTAGCCAGCACTACAGC
TAAGGCTATGGAGCAAATGGCTGAATCGAGTGAGCAAGCAGCAGAGGCCATGGAGGTTGCTAGTCAGGCTAGACAAATGGTGCAAGCGATGAGAACC
ATTGGGACTCATCCTAGCTCCAGTGCTGGTCTGAAAAATGATCTTCTTGAAAATTTGCAGGCCTATCAGAAACGAATGGGGGTGCAGATGCAACGGT
TCAAGTGATCCTCTCACTATTGCCGCAAATATCATTGGGATCTTGCACTTGACATTGTGGATTCTTGATCGTCTTTTTTTCAAATGCATTTACCGTC
GCTTTAAATACGGACTGAAAGGAGGGCCTTCTACGGAAGGAGTGCCAAAGTCTATGAGGGAAGAATATCGAAAGGAACAGCAGAGTGCTGTGGATGC
TGACGATGGTCATTTTGTCAGCATAGAGCTGGAGTAAAAAACTACCTTGTTTCTACT

## Claims

1. An isolated nucleic acid molecule comprising an influenza A virus nuclear export protein (NEP) gene wherein the codon for amino acid no. 20 of the NEP gene, glutamine, has been changed to encode for arginine, histidine, lysine, asparagine, aspartic acid, or glutamic acid

2. Nucleic acid molecule according to claim 1, wherein the codon for amino acid no. 20 of the NEP gene, glutamine, has been changed to encode for arginine.

3. Nucleic acid molecule according to claim 1 or 2, further comprising an influenza NS1 gene.

4. Nucleic acid molecule according to any one of claims 1 to 3, further comprising an expression promoter, a terminator sequence, a heterologous gene for expression, siRNA, short regulatory nucleotide/RNA sequences, or combinations thereof.

5. Nucleic acid molecule according to any one of claims 1 to 4, further comprising a heterologous gene for expression, wherein the heterologous gene for expression is 5' to the NEP gene and wherein the heterologous gene for expression preferably encodes for a therapeutic protein, especially a vaccination antigen, or a cytokine, especially IL-2, IL-24, IL-15, IL-12, GM-CSF, Antigens, TBc Antigens, or Luciferase.

6. Influenza A virus comprising an influenza A virus nuclear export protein (NEP) gene wherein the codon for amino acid no. 20 of the NEP gene, glutamine, has been changed to encode for arginine, histidine, lysine, asparagine, aspartic acid, glutamic acid.

**7.** Influenza A virus according to claim 6, wherein the codon for amino acid no. 20 of the NEP gene, glutamine, has been changed to encode for arginine.

**8.** Influenza A virus according to claim 6 or 7, further comprising codon changes in the genes compared to the influenza virus A/PR/8/34 (H1N1), preferably in the NS1 gene, the PB2 gene, the HA gene or combinations thereof, especially wherein

- the codon for amino acid no. 368 of the NS1 gene, tyrosine, has been changed to encode for a different amino acid at this position in the NS1 gene,
- the codon for amino acid no. 535 of the PB2 gene, methionine, has been changed to encode for a different amino acid at this position in the PB2 gene,
- the codon for amino acid no. 479 of the HA gene, glycine, has been changed to encode for a different amino acid at this position in the HA gene,
- or combinations thereof.

**9.** Influenza A virus according to any one of claims 6 to 8, further comprising the following codon changes:

- the codon for amino acid no. 368 of the NS1 gene, tyrosine, has been changed to encode for histidine at this position in the NS1 gene,
- the codon for amino acid no. 535 of the PB2 gene, methionin, has been changed to encode for isoleucine at this position in the PB2 gene, and
- the codon for amino acid no. 479 of the HA gene, glycine, has been changed to encode for a arginine at this position in the HA gene.

**10.** Influenza A virus according to any one of claims 6 to 9, further comprising a heterologous gene for expression, preferably encoding for a therapeutic protein, especially a vaccination antigen or a cytokine,.

**11.** Isolated nucleic acid according to any one of claims 1 to 5 or influenza A virus according to any one of claims 6 to 10 for use as a vaccine.

**12.** Isolated nucleic acid according to any one of claims 1 to 5 or influenza A virus according to any one of claims 6 to 10 for use in oncolytic tumour therapy.

**13.** Use of an isolated nucleic acid according to any one of claims 1 to 5 or influenza A virus according to any one of claims 6 to 10 for the expression of a heterologous gene.

**14.** Use of an isolated nucleic acid according to any one of claims 1 to 5 or influenza A virus according to any one of claims 6 to 10 for diagnosis in virology.

**15.** An isolated nucleic acid molecule comprising an influenza B or influenza C virus nuclear export protein (NEP) gene wherein the codon for amino acid no. 18 of the NEP gene of influenza B, alanine, has been changed, or wherein the codon for amino acid no. 104 of the NEP gene of influenza C, serine, has been changed.

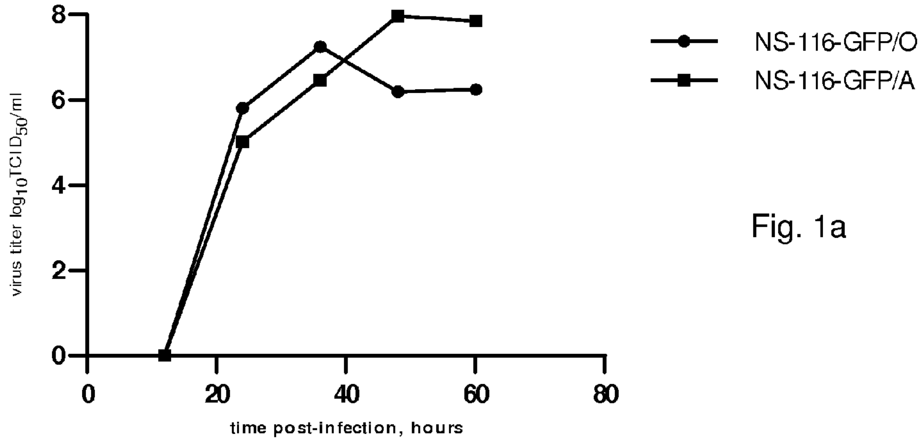


Fig. 1a

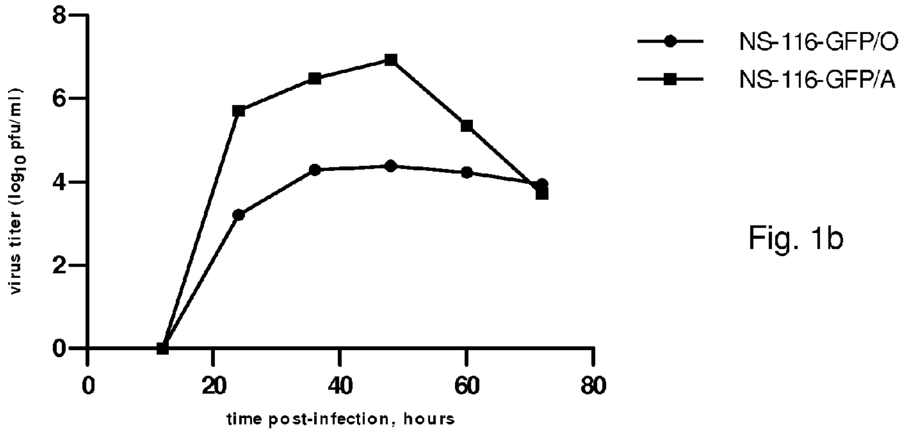


Fig. 1b

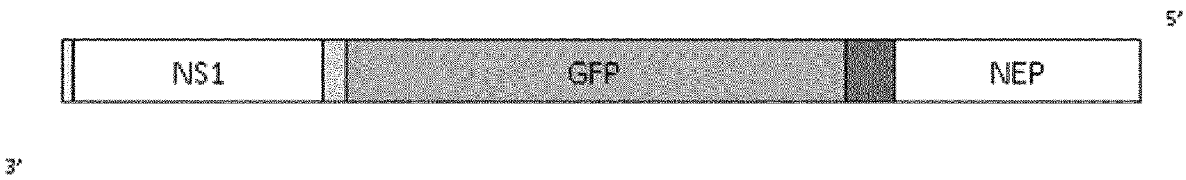


Fig. 2

Fig. 3a

NS-116-GFP/O NS-116-GFP/A

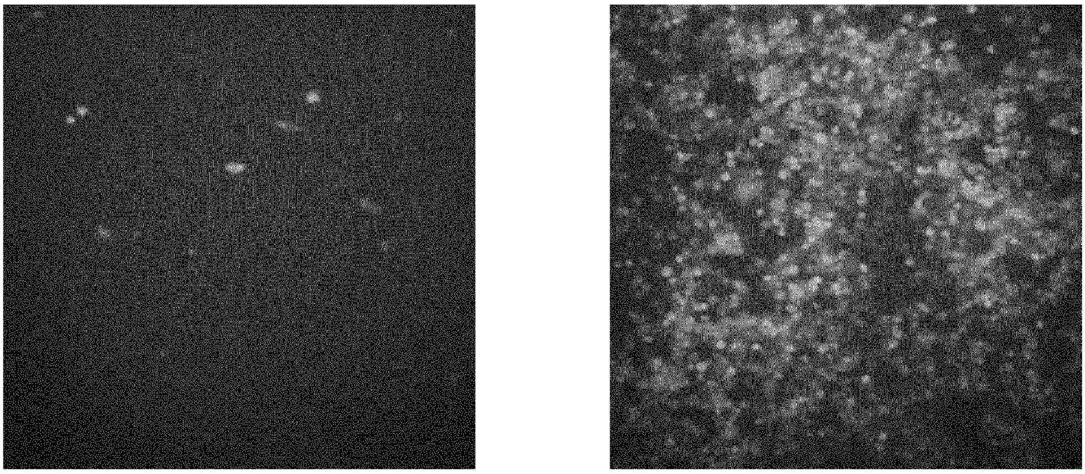

Fig. 3b

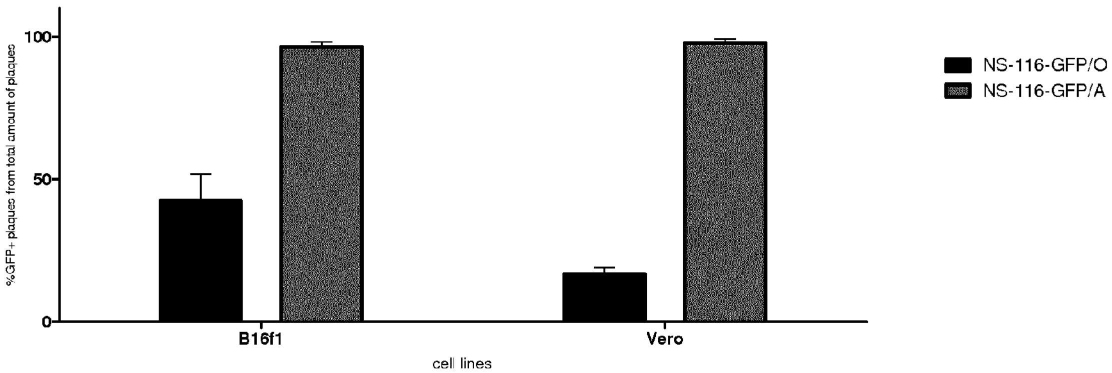

Fig. 4a

a

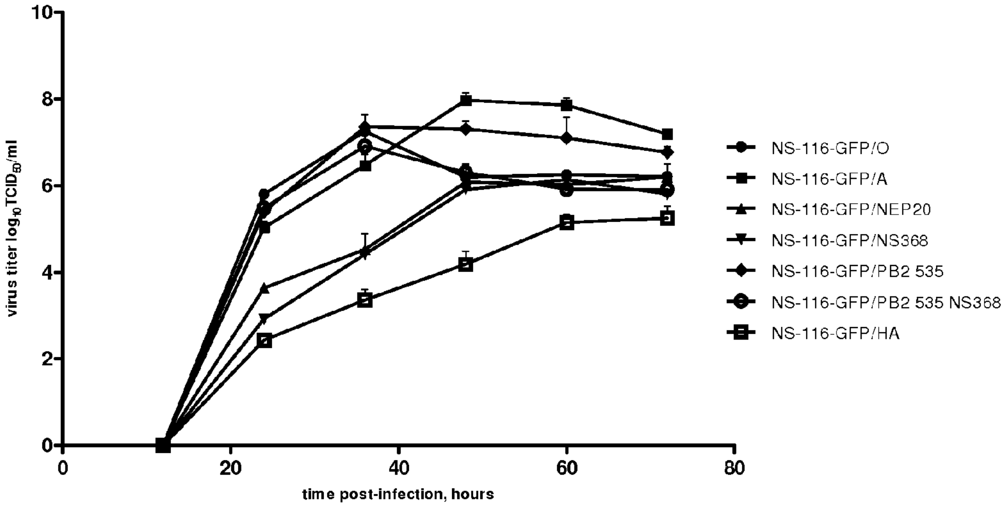


b

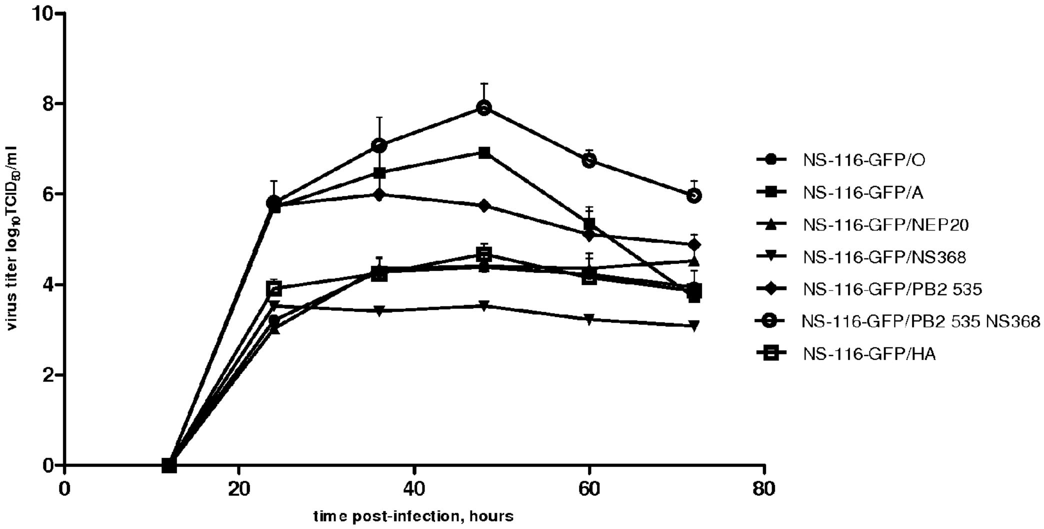


Fig. 4b

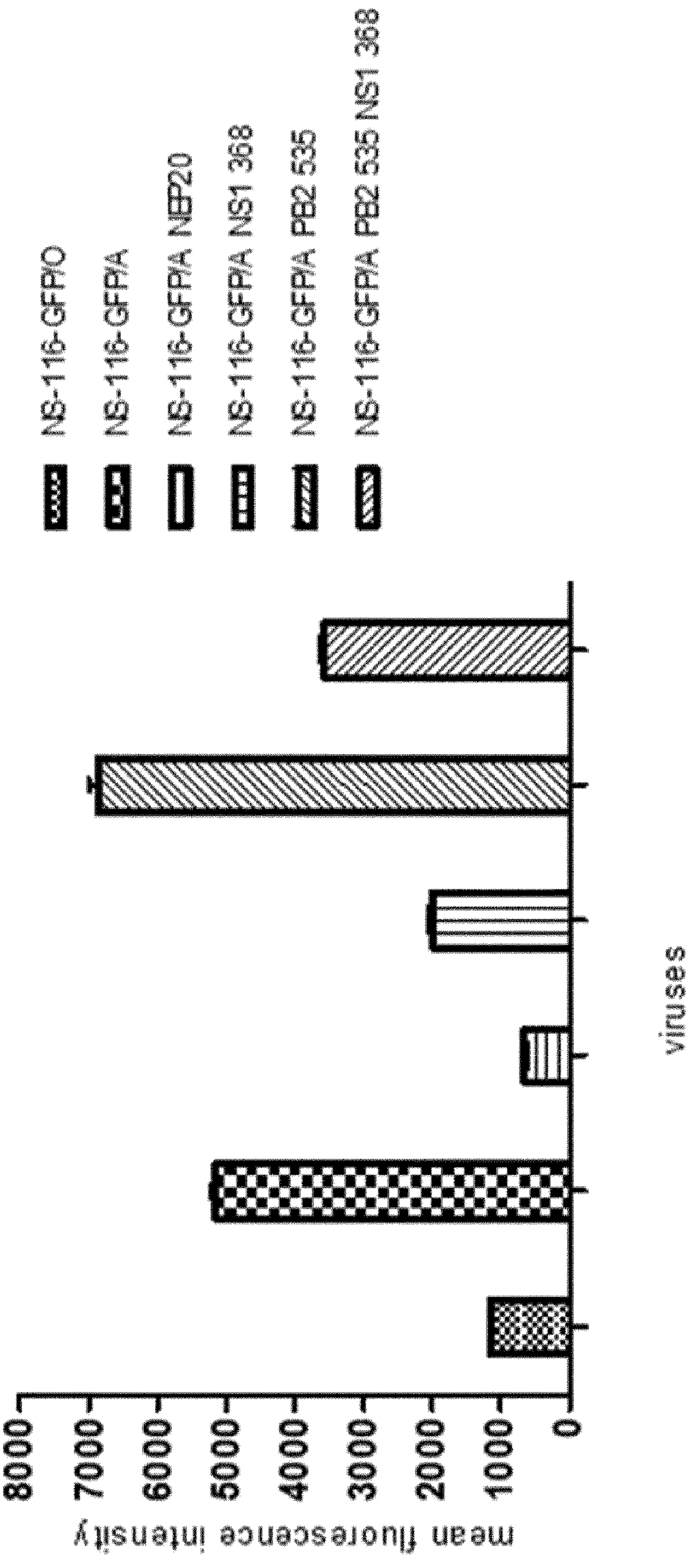
mean fluorescence intensity
8000
7000
6000
5000
4000
3000
2000
1000
0
viruses
NS-116-GFP/O
NS-116-GFP/A
NS-116-GFP/A NEP20
NS-116-GFP/A NS1 368
NS-116-GFP/A PB2 535
NS-116-GFP/A PB2 535 NS1 368

Fig. 5

Fig. 6a

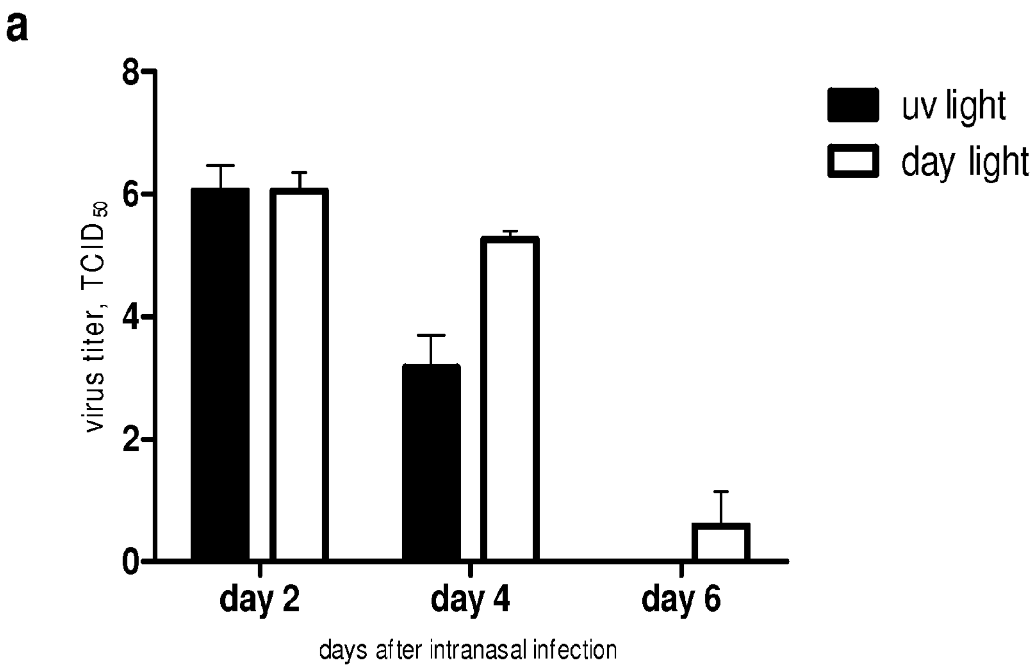
a
8
6
4
2
0
virus titer, TCID50
uv light
day light
day 2
day 4
day 6
days after intranasal infection

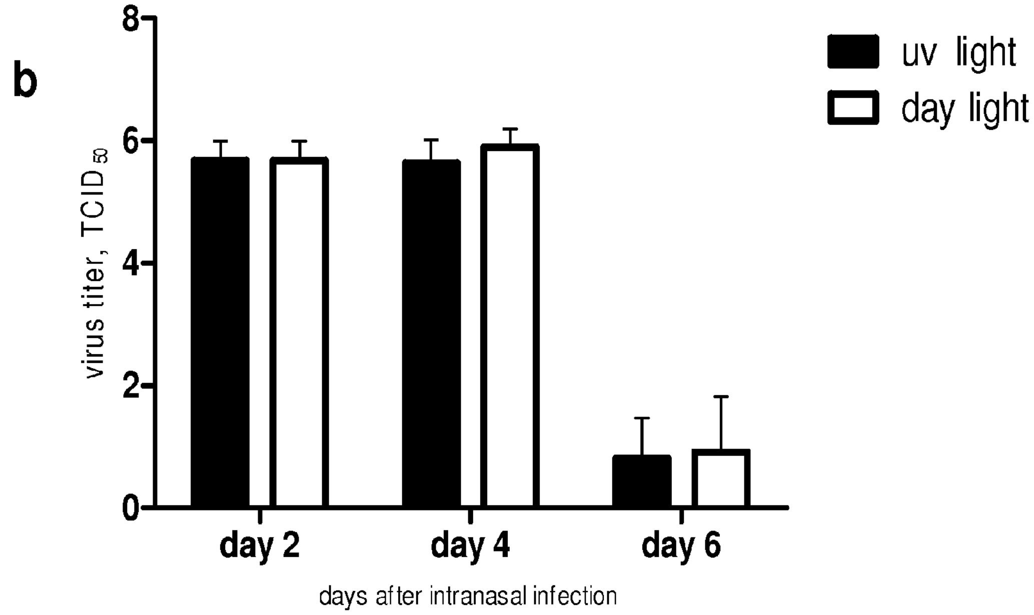
b
8
6
4
2
0
virus titer, TCID50
uv light
day light
day 2
day 4
day 6
days after intranasal infection

Fig. 6b

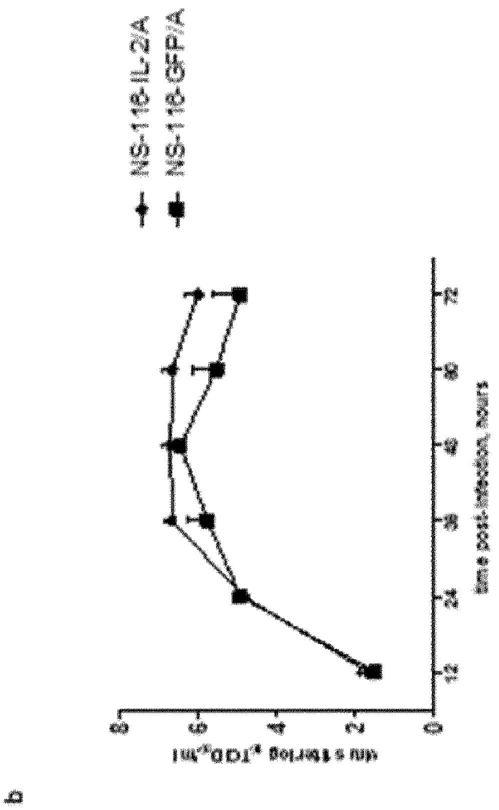


Fig. 8b

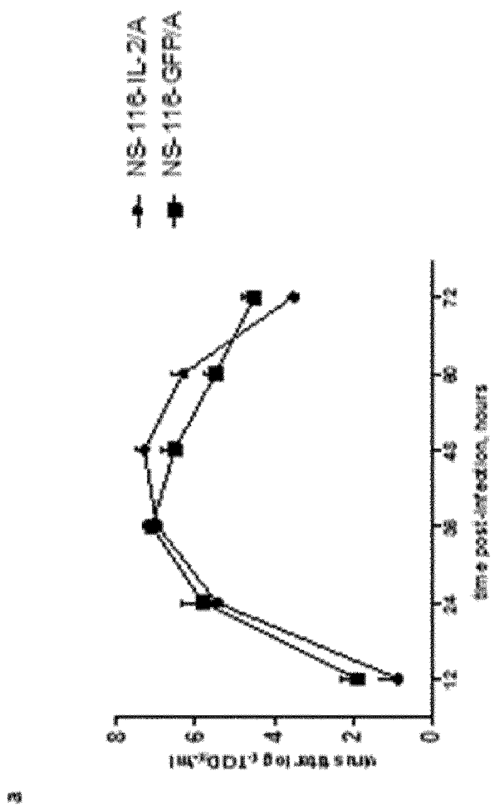


Fig. 8a

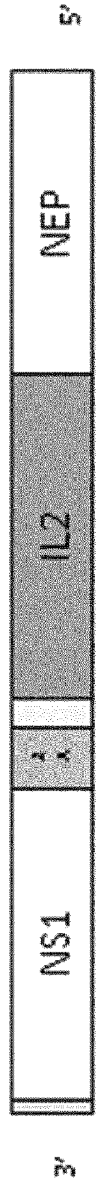


Fig. 7

Fig. 9a

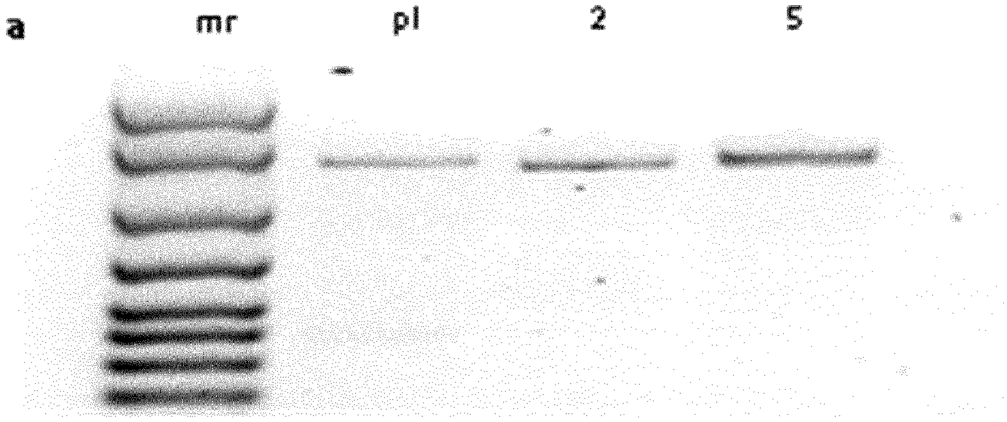
a
mr
pl
2
5

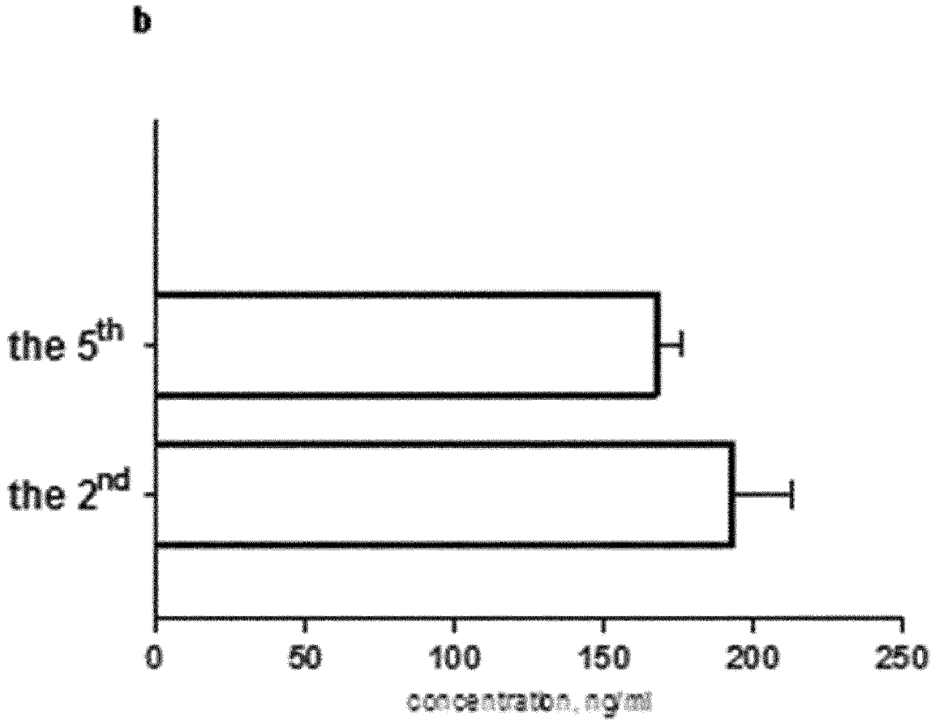
b
the 5th
the 2nd
0
50
100
150
200
250
concentration, ng/ml

Fig. 9b

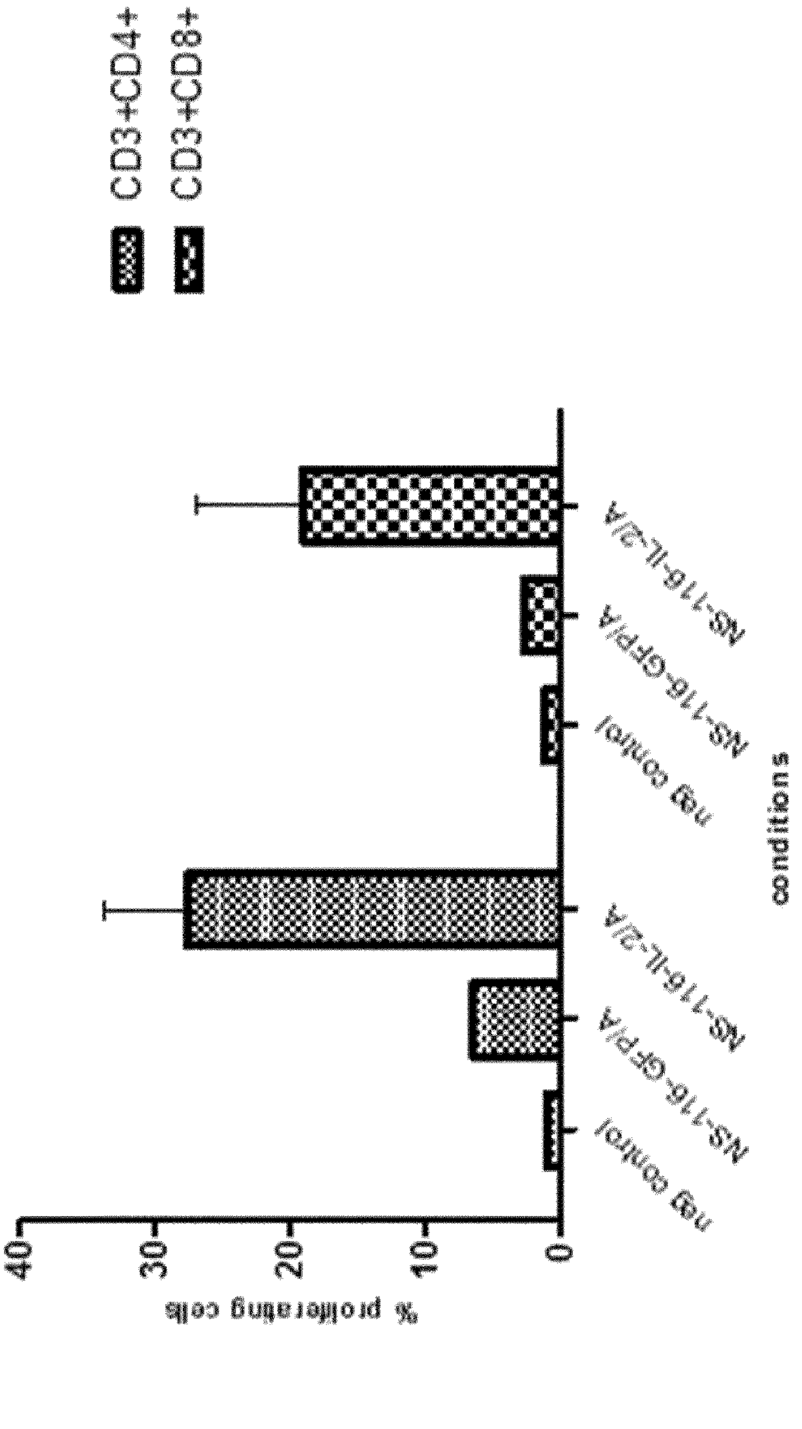
CD3+CD4+
CD3+CD8+
% proliferating cells
0
10
20
30
40
neg control
NS-116-GFP/A
NS-116-IL-2/A
conditions

Fig. 10

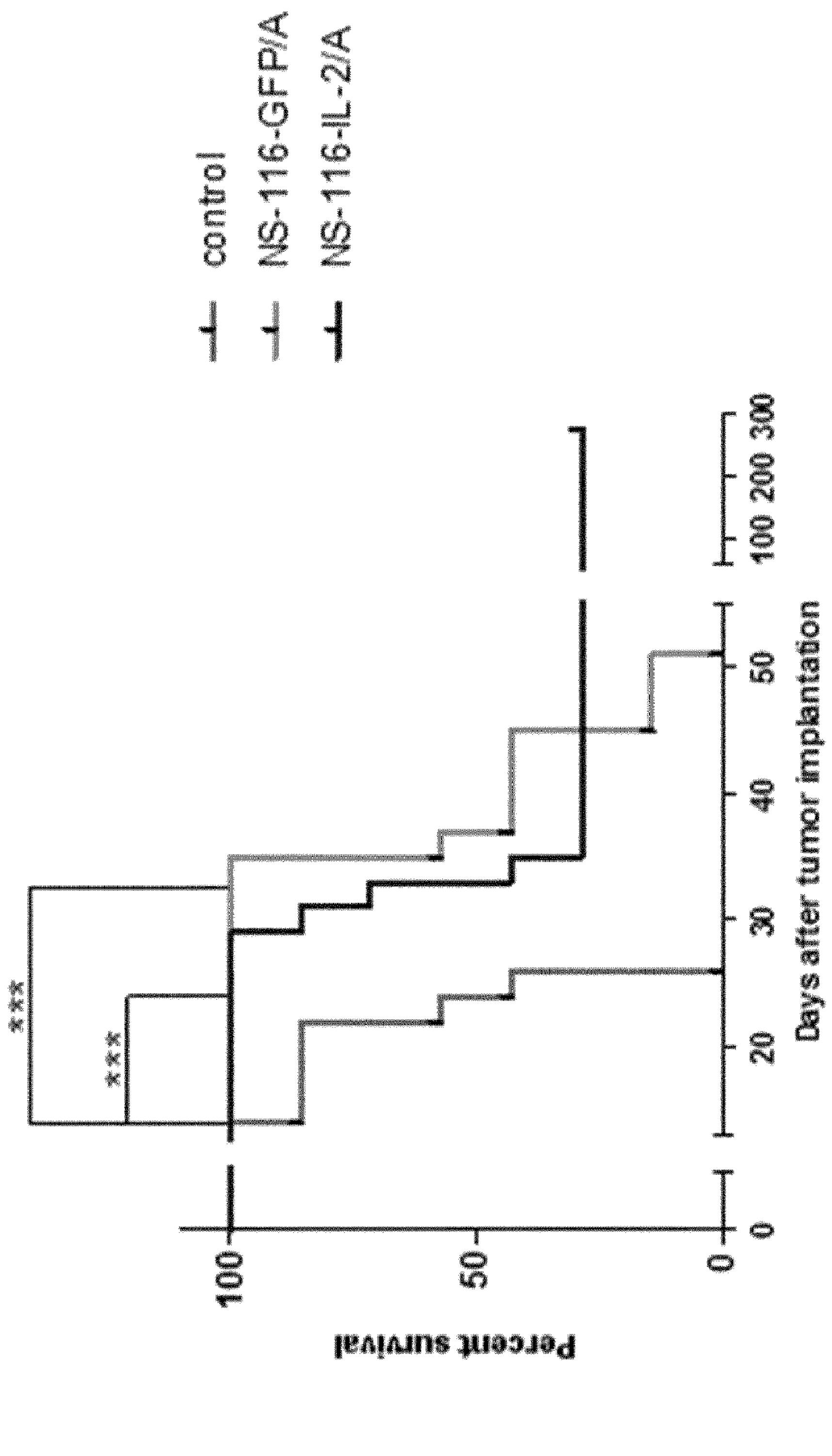
Percent survival
100
50
0
0
20
30
40
50
100 200 300
Days after tumor implantation
***
***
control
NS-116-GFP/A
NS-116-IL-2/A

Fig. 11

Fig. 12

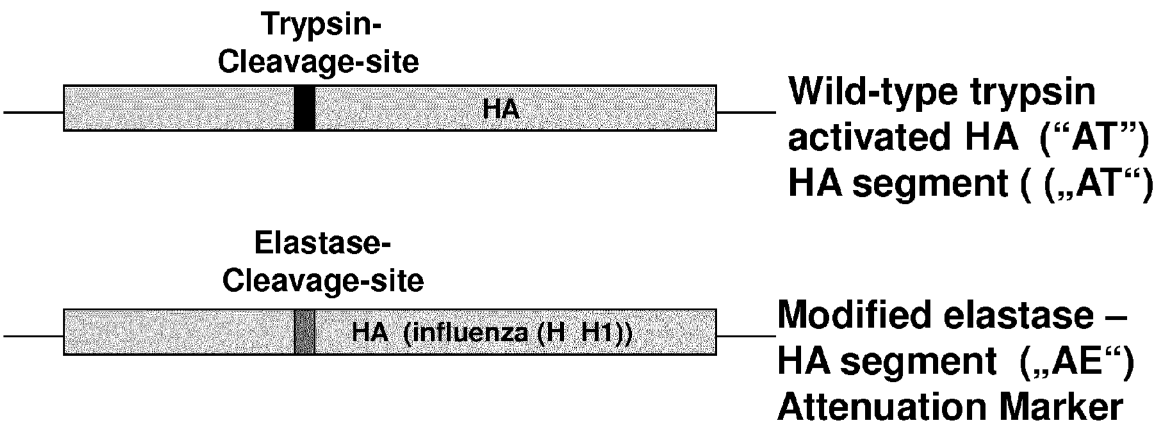
Trypsin-
Cleavage-site
HA
Wild-type trypsin
activated HA (“AT”)
HA segment ( („AT“)
Elastase-
Cleavage-site
HA (influenza (H H1))
Modified elastase –
HA segment („AE“)
Attenuation Marker

Fig. 13a

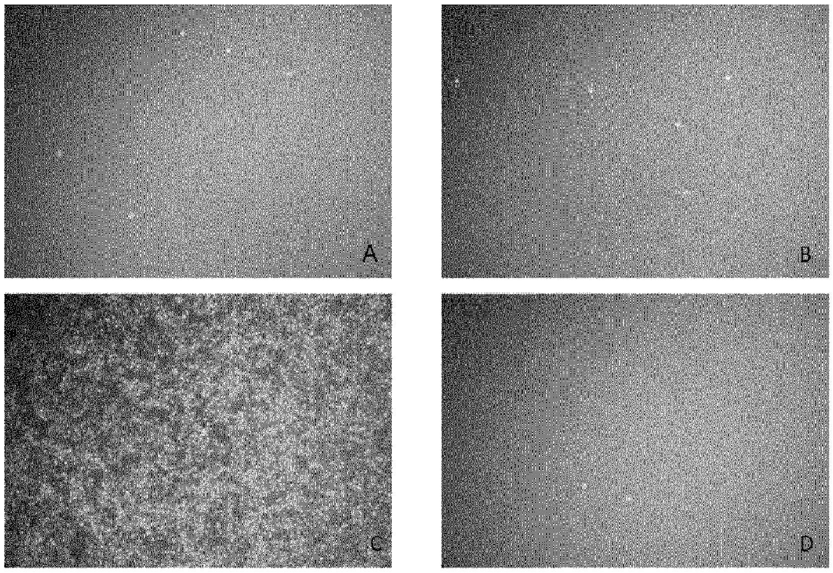


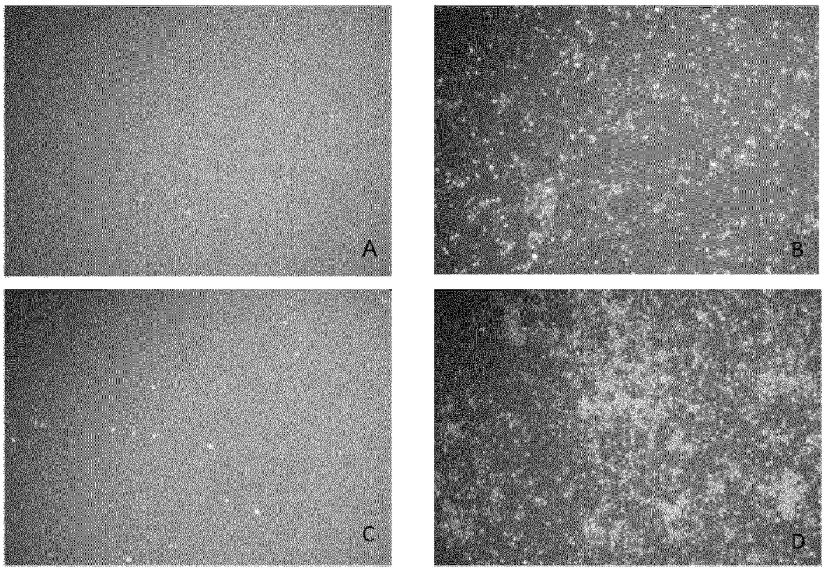


Fig. 13b

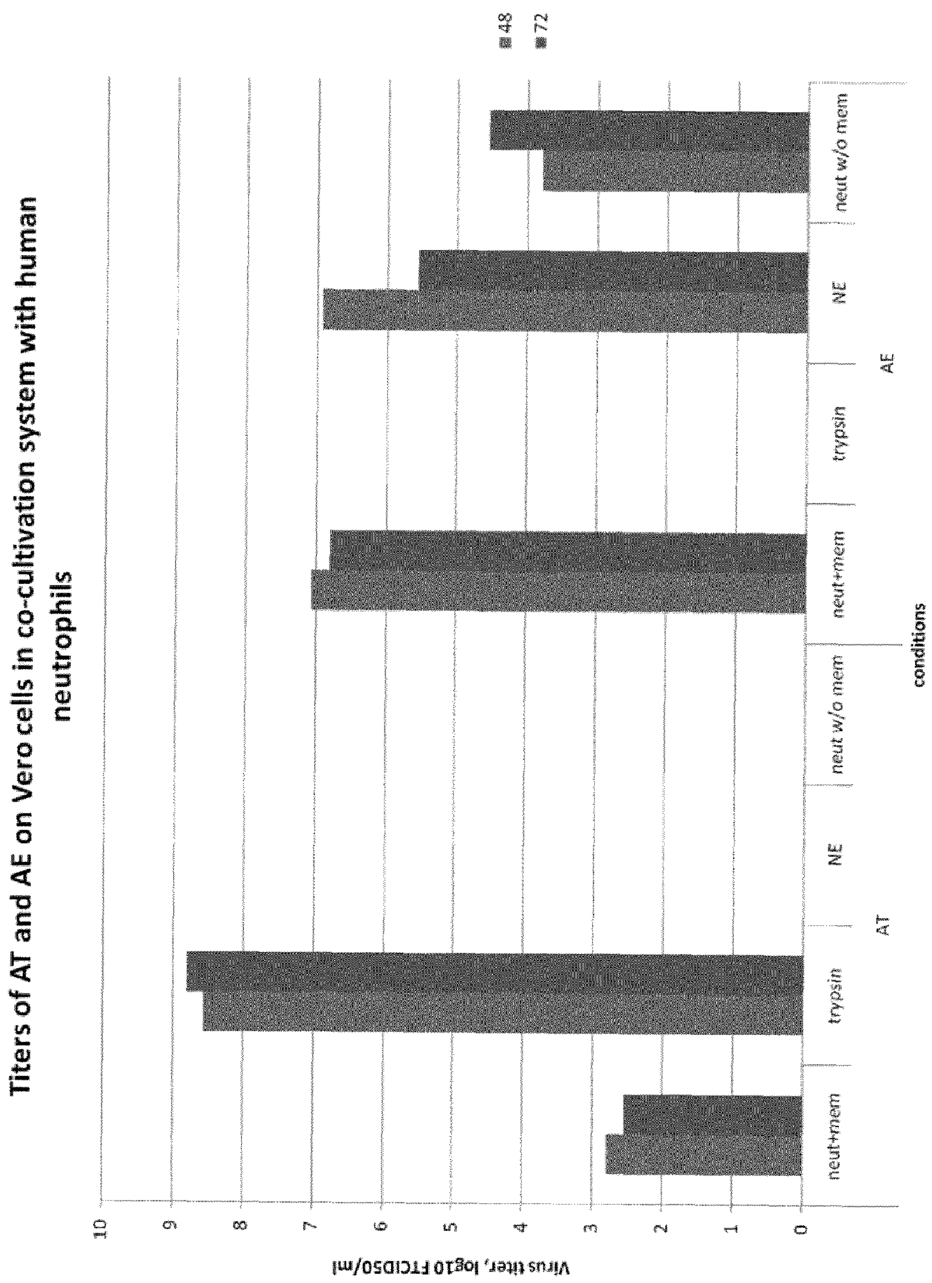
Titers of AT and AE on Vero cells in co-cultivation system with human neutrophils
Virus titer, log10 FTCID50/ml
0
1
2
3
4
5
6
7
8
9
10
neut+mem
trypsin
NE
neut w/o mem
neut+mem
trypsin
NE
neut w/o mem
AT
AE
conditions
48
72

Fig. 14

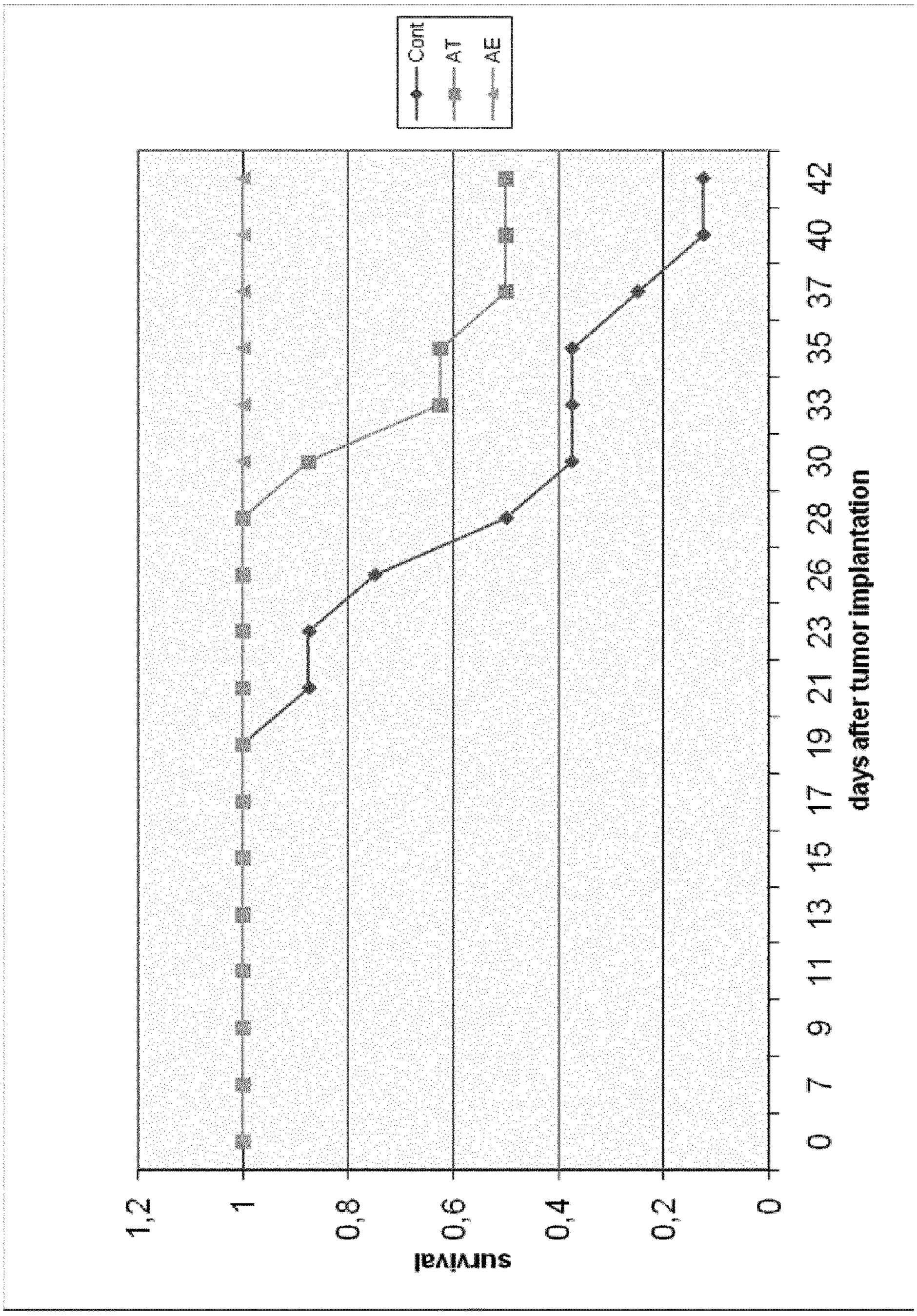
survival
1,2
1
0,8
0,6
0,4
0,2
0
0
7
9
11
13
15
17
19
21
23
26
28
30
33
35
37
40
42
days after tumor implantation
Cont
AT
AE

Fig. 15

Europäisches Patentamt
European Patent Office
Office européen des brevets

# EUROPEAN SEARCH REPORT

Application Number
EP 12 18 4041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>13 November 2007 (2007-11-13), "SubName: Full=Nonstructural protein 2;", XP002688474, retrieved from EBI accession no. UNIPROT:A8C0M8 Database accession no. A8C0M8 * NS2 protein having the substitution Q20R.; sequence * | 1,2 | INV. C07K14/11 |
| X | -& CHEUNG C L ET AL: "Establishment of influenza a virus (H6N1) in minor poultry species in southern China", JOURNAL OF VIROLOGY, vol. 81, no. 19, October 2007 (2007-10), pages 10402-10412, XP002688475, ISSN: 0022-538X * the whole document *<br>----- | 3-8, 10-14 | |
| X | DATABASE UniProt [Online]<br><br>13 November 2007 (2007-11-13), "SubName: Full=Nonstructural protein 2;", XP002688476, retrieved from EBI accession no. UNIPROT:A8CC92 Database accession no. A8CC92 * NS2 protein having the substitution Q20R.; sequence *<br>-/-- | 1,2 | TECHNICAL FIELDS SEARCHED (IPC)<br>C07K |

~~The present search report has been drawn up for all claims~~

2

| Place of search | Date of completion of the search | Examiner |
|---|---|---|
| Munich | 4 December 2012 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 4041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | -& K. M. XU ET AL: "The Genesis and Evolution of H9N2 Influenza Viruses in Poultry from Southern China, 2000 to 2005", JOURNAL OF VIROLOGY, vol. 81, no. 19, 25 July 2007 (2007-07-25), pages 10389-10401, XP055046290, ISSN: 0022-538X, DOI: 10.1128/JVI.00979-07 * the whole document * ----- | 3-8, 10-14 | |
| A,D | IWATSUKI-HORIMOTO K ET AL: "GENERATION OF INFLUENZA A VIRUS NS2 (NEP) MUTANTS WITH AN ALTERED NUCLEAR EXPORT SIGNAL SEQUENCE", JOURNAL OF VIROLOGY, THE AMERICAN SOCIETY FOR MICROBIOLOGY, US, vol. 78, no. 18, 1 September 2004 (2004-09-01), pages 10149-10155, XP009042802, ISSN: 0022-538X, DOI: 10.1128/JVI.78.18.10149-10155.2004 * the whole document * ----- | 1-14 | TECHNICAL FIELDS SEARCHED (IPC) |
| A,D | KITTEL CHRISTIAN ET AL: "Generation of an influenza A virus vector expressing biologically active human interleukin-2 from the NS gene segment", JOURNAL OF VIROLOGY, THE AMERICAN SOCIETY FOR MICROBIOLOGY, US, vol. 79, no. 16, 1 August 2005 (2005-08-01), pages 10672-10677, XP002414818, ISSN: 0022-538X, DOI: 10.1128/JVI.79.16.10672-10677.2005 * the whole document * ----- | 1-14 | |
| | -/-- | | |

~~The present search report has been drawn up for all claims~~

2

| Place of search | Date of completion of the search | Examiner |
|---|---|---|
| Munich | 4 December 2012 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 18 4041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | B. MANICASSAMY ET AL: "Analysis of in vivo dynamics of influenza virus infection in mice using a GFP reporter virus", PROCEEDINGS OF THE NATIONAL ACADEMY OF SCIENCES, vol. 107, no. 25, 7 June 2010 (2010-06-07), pages 11531-11536, XP055046225, ISSN: 0027-8424, DOI: 10.1073/pnas.0914994107<br>* the whole document *<br>----- | 1-14 | |
| A,D | M. WOLSCHEK ET AL: "Establishment of a Chimeric, Replication-Deficient Influenza A Virus Vector by Modulation of Splicing Efficiency", JOURNAL OF VIROLOGY, vol. 85, no. 5, 22 December 2010 (2010-12-22), pages 2469-2473, XP055046389, ISSN: 0022-538X, DOI: 10.1128/JVI.01650-10<br>* the whole document *<br>----- | 1-14 | TECHNICAL FIELDS SEARCHED (IPC) |
| A,D | WO 2008/140621 A2 (SINAI SCHOOL MEDICINE [US]; WOO SAVIO L C [US]; EBERT OLIVER [US]; GAR) 20 November 2008 (2008-11-20)<br>* the whole document *<br>----- | 1-14 | |

~~The present search report has been drawn up for all claims~~

2

| Place of search | Date of completion of the search | Examiner |
|---|---|---|
| Munich | 4 December 2012 | Rojo Romeo, Elena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 12 18 4041

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 12 18 4041

The Search Division considers that the present European patent application does not comply with the requirements of unity of invention and relates to several inventions or groups of inventions, namely:

1. claims: 1-14(partially)

Searched claims

1.1. claims: 1-14(partially)

An isolated nucleic acid molecule comprising an influenza A virus nuclear export protein (NEP) gene wherein the codon for amino acid no. 20 of the NEP gene, glutamine, has been changed to encode for arginine; corresponding influenza virus; embodiments thereof.

1.2. claims: 1-14(partially)

An isolated nucleic acid molecule comprising an influenza A virus nuclear export protein (NEP) gene wherein the codon for amino acid no. 20 of the NEP gene, glutamine, has been changed to encode for histidine; corresponding influenza virus; embodiments thereof.

1.3. claims: 1-14(partially)

An isolated nucleic acid molecule comprising an influenza A virus nuclear export protein (NEP) gene wherein the codon for amino acid no. 20 of the NEP gene, glutamine, has been changed to encode for lysine; corresponding influenza virus; embodiments thereof.

1.4. claims: 1-14(partially)

An isolated nucleic acid molecule comprising an influenza A virus nuclear export protein (NEP) gene wherein the codon for amino acid no. 20 of the NEP gene, glutamine, has been changed to encode for asparagine; corresponding influenza virus; embodiments thereof.

1.5. claims: 1-14(partially)

An isolated nucleic acid molecule comprising an influenza A virus nuclear export protein (NEP) gene wherein the codon for amino acid no. 20 of the NEP gene, glutamine, has been changed to encode for aspartic acid; corresponding influenza virus; embodiments thereof.

1.6. claims: 1-14(partially)

An isolated nucleic acid molecule comprising an influenza A virus nuclear export protein (NEP) gene wherein the codon for amino acid no. 20 of the NEP gene, glutamine, has been changed to encode for glutamic acid; corresponding influenza virus; embodiments thereof.

Europäisches Patentamt
European Patent Office
Office européen des brevets

# LACK OF UNITY OF INVENTION SHEET B

Application Number
EP 12 18 4041

The Search Division considers that the present European patent application does not comply with the requirements of unity of invention and relates to several inventions or groups of inventions, namely:

---

2. claim: 15(partially)

An isolated nucleic acid molecule comprising an influenza B virus nuclear export protein (NEP) gene wherein the codon for amino acid no. 18 of the NEP gene of influenza B, alanine, has been changed.

---

3. claim: 15(partially)

An isolated nucleic acid molecule comprising an influenza C virus nuclear export protein (NEP) gene wherein the codon for amino acid no. 104 of the NEP gene of influenza C, serine, has been changed.

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO. EP 12 18 4041

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2012

| Patent document cited in search report | Publication date | Patent family member(s) | Publication date |
|---|---|---|---|
| WO 2008140621 A2 | 20-11-2008 | US 2010178684 A1 | 15-07-2010 |
| | | WO 2008140621 A2 | 20-11-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011044561 A1
- WO 2011014504 A1
- WO 2001004333 A1
- WO 2003072725 A2
- WO 2008156778 A2
- WO 2008048306 A2
- WO 2012088428 A1
- WO 2008140621 A2


### Non-patent literature cited in the description


- **WOLSCHEK et al.** *J. Virol.,* 2011, vol. 85, 2469-2473
- **IWATSUKI-HORIMOTO et al.** *J. Virol.,* 2004, vol. 78, 10149-10155
- **RIMMELZWAAN et al.** *Vaccine,* 2011, vol. 29, 3424-3430
- **HOFFMANN et al.** *PNAS,* 2000, vol. 97, 6108-6113
- **KITTEL et al.** *J. Virol.,* 2005, vol. 79, 10672-10677
- **MANICASSAMY et al.** *PNAS,* 2010, vol. 107, 11531-11536
- **STECH et al.** *Nature Medicine,* 2005, vol. 11, 683-689

SEQUENCE LISTING

<110> Medizinische Universität Wien

<120> Influenza Virus

<130> R 62447

<160> 8

<170> PatentIn version 3.5

<210> 1
<211> 1413
<212> DNA
<213> Influenza A virus

<400> 1
agcgaaagca ggagtttaaa atgaatccaa atcagaaaat aataaccatt ggatcaatct 60

gtctggtagt cggactaatt agcctaatat tgcaaatagg gaatataatc tcaatatgga 120

ttagccattc aattcaaact ggaagtcaaa accatactgg aatatgcaac caaaacatca 180

ttacctataa aaatagcacc tgggtaaagg acacaacttc agtgatatta accggcaatt 240

catctctttg tcccatccgt gggtgggcta tatacagcaa agacaatggc ataagaattg 300

gttccaaagg agacgttttt gtcataagag agccctttat ttcatgttct cacttggaat 360

gcaggacctt ttttctgacc caaggtgcct tactgaatga caagcattca agtgggactg 420

ttaaggacag aagcccttat agggccttaa tgagctgccc tgtcggtgaa gctccgtccc 480

cgtacaattc aagatttgaa tcggttgctt ggtcagcaag tgcatgtcat gatggcatgg 540

gctggctaac aatcggaatt tcaggtccag ataatggagc agtggctgta ttaaaataca 600

acggcataat aactgaaacc ataaaaagtt ggaggaagaa aatattgagg acacaagagt 660

ctgaatgtgc ctgtgtaaat ggttcatgtt ttactataat gactgatggc ccgagtgatg 720

ggctggcctc atacaaaatt ttcaagatcg aaaaggggaa ggttactaaa tcaatagagt 780

tgaatgcacc taattctcac tatgaggaat gttcctgtta ccctgatacc ggcaaagtga 840

tgtgtgtgtg cagagacaat tggcatggtt cgaaccggcc atgggtgtct ttcgatcaaa 900

acctggatta tcaaatagga tacatctgca gtggggtttt cggtgacaac ccgcgtcccg 960

aagatggaac aggcagctgt ggtccagtgt atgttgatgg agcaaacgga gtaaagggat 1020

tttcatatag gtatggtaat ggtgtttgga taggaaggac caaaagtcac agttccagac 1080

atgggtttga gatgatttgg gatcctaatg gatggacaga gactgatagt aagttctctg 1140

tgaggcaaga tgttgtggca atgactgatt ggtcagggta tagcggaagt ttcgttcaac 1200

atcctgagct gacagggcta gactgtatga ggccgtgctt ctgggttgaa ttaatcaggg 1260

gacgacctaa agaaaaaaca atctggacta gtgcgagcag catttctttt tgtggcgtga 1320

atagtgatac tgtagattgg tcttggccag acggtgctga gttgccattc agcattgaca 1380

agtagtctgt tcaaaaaact ccttgtttct act 1413

<210> 2
<211> 1565
<212> DNA
<213> Influenza A virus

<400> 2
agcaaaagca gggtagataa tcactcactg agtgacatca aaatcatggc gtcccaaggc 60

accaaacggt cttacgaaca gatggagact gatggagaac gccagaatgc cactgaaatc 120

agagcatccg tcggaaaaat gattggtgga attggacgat tctacatcca aatgtgcacc 180

gaactcaaac tcagtgatta tgagggacgg ttgatccaaa acagcttaac aatagagaga 240

atggtgctct ctgcttttga cgaaaggaga aataaatacc tggaagaaca tcccagtgcg 300

gggaaagatc ctaagaaaac tggaggacct atatacagga gagtaaacgg aaagtggatg 360

agagaactca tcctttatga caaagaagaa ataaggcgaa tctggcgcca agctaataat 420

ggtgacgatg caacggctgg tctgactcac atgatgatct ggcattccaa tttgaatgat 480

gcaacttatc agaggacaag agctcttgtt cgcaccggaa tggatcccag gatgtgctct 540

ctgatgcaag gttcaactct ccctaggagg tctggagccg caggtgctgc agtcaaagga 600

gttggaacaa tggtgatgga attggtcagg atgatcaaac gtgggatcaa tgatcggaac 660

ttctggaggg gtgagaatgg acgaaaaaca agaattgctt atgaaagaat gtgcaacatt 720

ctcaaaggga aatttcaaac tgctgcacaa aaagcaatga tggatcaagt gagagagagc 780

cggaacccag ggaatgctga gttcgaagat ctcacttttc tagcacggtc tgcacttata 840

ttgagagggt cggttgctca caagtcctgc ctgcctgcct gtgtgtatgg acctgccgta 900

gccaatgggt acgactttga aagagaggga tactctctag tcggaataga ccctttcaga 960

ctgcttcaaa acagccaagt gtacagccta atcagaccaa atgagaatcc agcacacaag 1020

agtcaactgg tgtggatggc atgccattct gccgcatttg aagatctaag agtattaagc 1080

ttcatcaaag ggacgaaggt gctcccaaga gggaagcttt ccactagagg agttcaaatt 1140

gcttccaatg aaaatatgga gactatggaa tcaagtacac ttgaactgag aagcaggtac 1200

tgggccataa ggaccagaag tggaggaaac accaatcaac agagggcatc tgcgggccaa 1260

atcagcatac aacctacgtt ctcagtacag agaaatctcc cttttgacag aacaaccatt 1320

atggcagcat tcaatgggaa tacagagggg agaacatctg acatgaggac cgaaatcata 1380

aggatgatgg aaagtgcaag accagaagat gtgtctttcc aggggcgggg agtcttcgag 1440

ctctcggacg aaaaggcagc gagcccgatc gtgccttcct ttgacatgag taatgaagga 1500

tcttatttct tcggagacaa tgcagaggag tacgacaatt aaagaaaaat acccttgttt 1560

ctact 1565

<210> 3
<211> 1668

<212> DNA
<213> Influenza A virus

<400> 3
agcaaaagca gggtgacaaa gacataatgg atccaaacac tgtgtcaagc tttcaggtag 60

attgctttct ttggcatgtc cgcaaacgag ttgcagacca agaactaggt gatgccccat 120

tccttgatcg gcttcgccga gatcagaaat ccctaagagg aaggggcagc accctcggtc 180

tggacatcga gacagccaca cgtgctggaa agcagatagt ggagcggatt ctgaaagaag 240

aatccgatga ggcacttaaa atgaccatgg cctctgtacc tgcgtcgcgt tacctaactg 300

acatgactct tgaggaaatg tcaagggact ggtccatgtc tttgtatcag aatggaccag 360

gcgatcatgg attaatggct agcaaaggag aagaactctt cactggagtt gtcccaattc 420

ttgttgaatt agatggtgat gttaacggcc acaaattctc tgtcagtgga gagggtgaag 480

gtgatgcaac atacggaaaa cttaccctga agttcatctg cactactggc aaactgcctg 540

ttccatggcc aacactagtc actactctgt gctatggtgt tcaatgcttt tcaagatacc 600

cggatcatat gaaacggcat gactttttca agagtgccat gcccgaaggt tatgtacagg 660

aaaggaccat cttcttcaaa gatgacggca actacaagac acgtgctgaa gtcaagtttg 720

aaggtgatac ccttgttaat agaatcgagt taaaaggtat tgacttcaag gaagatggca 780

acattctggg acacaaattg gaatacaact ataactcaca caatgtatac atcatggcag 840

acaaacaaaa gaatggaatc aaagtgaact tcaagacccg ccacaacatt gaagatggaa 900

gcgttcaact agcagaccat tatcaacaaa atactccaat tggcgatggc cctgtccttt 960

taccagacaa ccattacctg tccacacaat ctgccctttc gaaagatccc aacgaaaaga 1020

gagaccacat ggtccttctt gagtttgtaa cagctgctgg gattacacat ggcatggatg 1080

aactgtacaa catcgatgga ggcggaggtg gagggcccgg ttaccggcac cggatccaga 1140

tatctgggcg gccgctcagc aagcttcgcg aattctgata agaacatcat actgaaagcg 1200

aacttcagtg tgatttttga ccggctggag actctaatat tgctaagggc tttcaccgaa 1260

gagggagcaa ttgttggcga aatttcacca ttgccttctc ttccaggaca tactgctgag 1320

gatgtcaaaa atgcggttgg agtcctcatc gggggacttg aaaggaatga taacacagtt 1380

cgagtctctg aaactctaca gagattcgct tggagaagca gtaatgagaa tgggagacct 1440

ccactcactc caaaacagaa acgagaaatg gcgggaacaa ttaggtcaga agtttgaaga 1500

aataagatgg ttgattgaag aagtgagaca caaactgaag ataacagaga atagttttga 1560

gcaaataaca tttatgcaag ccttacatct attgcttgaa gtggagcaag agataagaac 1620

tttctcgttt cagcttattt agtaataaaa aacacccttg tttctact 1668

<210> 4
<211> 2233
<212> DNA
<213> Influenza A virus

<400> 4
agcgaaagca ggtactgatc caaaatggaa gattttgtgc gacaatgctt caatccgatg 60
attgtcgagc ttgcggaaaa aacaatgaaa gagtatgggg agaacctgaa aatcgaaaca 120
aacaaatttg cagcaatatg cactcacttg gaagtatgct tcatgtattc agattttcac 180
ttcatcaatg agcaaggcga gtcaataatc gtagaacttg gtgatccaaa tgcacttttg 240
aagcacagat ttgaaataat cgagggaaga gatcgcacaa tggcctggac agtagtaaac 300
agtatttgca acactacagg ggctgagaaa ccaaagttcc taccagattt gtatgattac 360
aaggagaata gattcatcga aattggagta acaaggagag aagttcacat atactatctg 420
gaaaaggcca ataaaattaa atctgagaaa acacacatcc acattttctc gttcactggg 480
gaagaaatgg ccacaaaggc agactacact ctcgatgaag aaagcagggc taggatcaaa 540
accagactat tcaccataag acaagaaatg gccagcagag gcctctggga ttcctttcgt 600
cagtccgaga gaggagaaga gacaattgaa gaaaggtttg aaatcacagg aacaatgcgc 660
aagcttgccg accaaagtct cccgccgaac ttctccagcc ttgaaaattt tagagcctat 720
gtggatggat tcgaaccgaa cggctacatt gagggcaagc tgtctcaaat gtccaaagaa 780
gtaaatgcta gaattgaacc tttttgaaa acaacaccac gaccacttag acttccgaat 840
gggcctctct gttctcagcg gtccaaattc ctgctgatgg atgccttaaa attaagcatt 900
gaggacccaa gtcatgaagg agagggaata ccgctatatg atgcaatcaa atgcatgaga 960
acattctttg gatggaagga acccaatgtt gttaaaccac acgaaaaggg aataaatcca 1020
aattatcttc tgtcatggaa gcaggtactg gcagaactgc aggacattga gaatgaggag 1080
aaaattccaa agactaaaat tatgaagaaa acaagtcagc taaagtgggc acttggtgag 1140
aacatggcac cagaaaaggt agactttgac gactgtaaag atgtaggtga tttgaagcaa 1200
tatgatagtg atgaaccaga attgaggtcg cttgcaagtt ggattcagaa tgagtttaac 1260
aaggcatgcg aactgacaga ttcaagctgg atagagctcg atgagattgg agaagatgtg 1320
gctccaattg aacacattgc aagcatgaga aggaattatt tcacatcaga ggtgtctcac 1380
tgcagagcca cagaatacat aatgaagggg gtgtacatca atactgcctt gcttaatgca 1440
tcttgtgcag caatggatga tttccaatta attccaataa taagcaagtg tagaactaag 1500
gagggaaggc gaaagaccaa cttgtatggt ttcatcataa aaggaagatc ccacttaagg 1560
aatgacaccg acgtggtaaa ctttgtgagc atggagtttt ctctcactga cccaagactt 1620
gaaccacata aatgggagaa gtactgtgtt cttgagatag gagatatgct tataagaagt 1680
gccataggcc aggtttcaag gcccatgttc ttgtatgtga gaacaaatgg aacctcaaaa 1740
attaaaatga aatggggaat ggagatgagg cgttgcctcc tccagtcact tcaacaaatt 1800
gagagtatga ttgaagctga gtcctctgtc aaagagaaag acatgaccaa agagttcttt 1860
gagaacaaat cagaaacatg gcccattgga gagtccccca aggagtgga ggaaagttcc 1920

attgggaagg tctgcaggac tttattagca aagtcggtat tcaacagctt gtatgcatct 1980

ccacaactag aaggattttc agctgaatca agaaaactgc ttcttatcgt tcaggctctt 2040

agggacaacc ttgaacctgg gacctttaat cttggggggc tatatgaagc aattgaggag 2100

tgcctgatta atgatccctg ggttttgctt aatgcttctt ggttcaactc cttccttaca 2160

catgcattga gttagttgtg gcagtgctac tatttgctat ccatactgtc caaaaaagta 2220

ccttgtttct act 2233

<210> 5
<211> 2341
<212> DNA
<213> Influenza A virus

<400> 5
agcgaaagca ggcaaaccat ttgaatggat gtcaatccga ccttactttt cttaaaagtg 60

ccagcacaaa atgctataag cacaactttc ccttatactg gagaccctcc ttacagccat 120

gggacaggaa caggatacac catggatact gtcaacagga cacatcagta ctcagaaaag 180

ggaagatgga caacaaacac cgaaactgga gcaccgcaac tcaacccgat tgatgggcca 240

ctgccagaag acaatgaacc aagtggttat gcccaaacag attgtgtatt ggaggcgatg 300

gctttccttg aggaatccca tcctggtatt tttgaaaact cgtgtattga aacgatggag 360

gttgttcagc aaacacgagt agacaagctg acacaaggcc gacagaccta tgactggact 420

ctaaatagaa accaacctgc tgcaacagca ttggccaaca caatagaagt gttcagatca 480

aatggcctca cggccaatga gtctggaagg ctcatagact tccttaagga tgtaatggag 540

tcaatgaaca aagaagaaat ggggatcaca actcattttc agagaaagag acgggtgaga 600

gacaatatga ctaagaaaat gataacacag agaacaatgg gtaaaaagaa gcagagattg 660

aacaaaagga gttatctaat tagagcattg accctgaaca caatgaccaa agatgctgag 720

agagggaagc taaaacggag agcaattgca accccaggga tgcaaataag gggtttgta 780

tactttgttg agacactggc aaggagtata tgtgagaaac ttgaacaatc agggttgcca 840

gttggaggca atgagaagaa agcaaagttg gcaaatgttg taaggaagat gatgaccaat 900

tctcaggaca ccgaactttc tttcaccatc actggagata acaccaaatg gaacgaaaat 960

cagaatcctc ggatgttttt ggccatgatc acatatatga ccagaaatca gcccgaatgg 1020

ttcagaaatg ttctaagtat tgctccaata atgttctcaa acaaaatggc gagactggga 1080

aaagggtata tgtttgagag caagagtatg aaacttagaa ctcaaatacc tgcagaaatg 1140

ctagcaagca tcgatttgaa atatttcaat gattcaacaa gaaagaagat tgaaaaaatc 1200

cgaccgctct taataggggg gactgcatca ttgagccctg gaatgatgat gggcatgttc 1260

aatatgttaa gcactgtatt aggcgtctcc atcctgaatc ttggacaaaa gagatacacc 1320

aagactactt actggtggga tggtcttcaa tcctctgacg attttgctct gattgtgaat 1380

gcacccaatc atgaagggat tcaagccgga gtcgacaggt tttatcgaac ctgtaagcta 1440

cttggaatca atatgagcaa gaaaaagtct tacataaaca gaacaggtac atttgaattc 1500

acaagttttt tctatcgtta tgggtttgtt gccaatttca gcatggagct tcccagtttt 1560

ggggtgtctg ggatcaacga gtcagcggac atgggtattg gagttactgt catcaaaaac 1620

aatatgataa acaatgatct tggtccagca acagctcaaa tggcccttca gttgttcatc 1680

aaagattaca ggtacacgta ccgatgccat agaggtgaca cacaaataca aacccgaaga 1740

tcatttgaaa taaagaaact gtgggagcaa acccgttcca aagctggact gctggtctcc 1800

gacggaggcc caaatttata caacattaga aatctccaca ttcctgaagt ctgcctaaaa 1860

tgggaattga tggatgagga ttaccagggg cgtttatgca acccactgaa cccatttgtc 1920

agccataaag aaattgaatc aatgaacaat gcagtgatga tgccagcaca tggtccagcc 1980

aaaaacatgg agtatgatgc tgttgcaaca acacactcct ggatccccaa aagaaatcga 2040

tccatcttga atacaagtca aagaggagta cttgaggatg aacaaatgta ccaaaggtgc 2100

tgcaatttat ttgaaaaatt cttccccagc agttcataca gaagaccagt cgggatatcc 2160

aatatggtgg aggctatggt ttccagagcc cgaattgatg cacggattga tttcgaatct 2220

ggaaggataa agaaagaaga gttcactgag atcatgaaga tctgttccac cattgaagag 2280

ctcagacggc aaaaatagtg aatttagctt gtccttcatg aaaaaatgcc ttgtttctac 2340

t 2341

<210> 6
<211> 2341
<212> DNA
<213> Influenza A virus

<400> 6
agcgaaagca ggtcaattat attcaatatg gaaagaataa aagaactaag aaatctaatg 60

tcgcagtctc gcacccgcga gatactcaca aaaaccaccg tggaccatat ggccataatc 120

aagaagtaca catcaggaag acaggagaag aacccagcac ttaggatgaa atggatgatg 180

gcaatgaaat atccaattac agcagacaag aggataacgg aaatgattcc tgagagaaat 240

gagcaaggac aaactttatg gagtaaaatg aatgatgccg gatcagaccg agtgatggta 300

tcacctctgg ctgtgacatg gtggaatagg aatggaccaa taacaaatac agttcattat 360

ccaaaaatct acaaaactta ttttgaaaga gtcgaaaggc taaagcatgg aacctttggc 420

cctgtccatt ttagaaacca agtcaaaata cgtcggagag ttgacataaa tcctggtcat 480

gcagatctca gtgccaagga ggcacaggat gtaatcatgg aagttgtttt ccctaacgaa 540

gtgggagcca ggatactaac atcggaatcg caactaacga taaccaaaga gaagaaagaa 600

gaactccagg attgcaaaat ttctcctttg atggttgcat acatgttgga gagagaactg 660

gtccgcaaaa cgagattcct cccagtggct ggtggaacaa gcagtgtgta cattgaagtg 720

ttgcatttga ctcaaggaac atgctgggaa cagatgtata ctccaggagg ggaagtgagg 780
aatgatgatg ttgatcaaag cctgattatt gctgctagga acatagtgag aagagctgca 840
gtatcagcag atccactagc atctttattg gagatgtgcc acagcacaca gattggtgga 900
attaggatgg tagacatcct taggcagaac ccaacagaag agcaagccgt ggatatatgc 960
aaggctgcaa tgggactgag aattagctca tccttcagtt ttggtggatt cacatttaag 1020
agaacaagcg gatcatcagt caagagagag gaagaggtgc ttacgggcaa tcttcaaaca 1080
ttgaagataa gagtgcatga gggatatgaa gagttcacaa tggttgggag aagagcaaca 1140
gccatactca gaaaagcaac caggagattg attcagctga tagtgagtgg gagagacgaa 1200
cagtcgattg ccgaagcaat aattgtggcc atggtatttt cacaagagga ttgtatgata 1260
aaagcagtca gaggtgatct gaatttcgtc aatagggcga atcagcgatt gaatcctatg 1320
catcaacttt taagacattt tcagaaggat gcgaaagtgc tttttcaaaa ttggggagtt 1380
gaacctatcg acaatgtgat gggaatgatt gggatattgc ccgacatgac tccaagcatc 1440
gagatgtcaa tgagaggagt gagaatcagc aaaatgggtg tagatgagta ctccagcacg 1500
gagagggtag tggtgagcat tgaccgtttt ttgagaatcc gggaccaacg aggaaatgta 1560
ctactgtctc ccgaggaggt cagtgaaaca cagggaacag agaaactgac aataacttac 1620
tcatcgtcaa taatgtggga gattaatggt cctgaatcag tgttggtcaa tacctatcaa 1680
tggatcatca gaaactggga aactattaaa attcagtggt cccagaaccc tacaatgcta 1740
tacaataaaa tggaatttga accatttcag tctttagtac ctaaggccat tagaggccaa 1800
tacagtgggt ttgtaagaac tctgttccaa caaatgaggg atgtgcttgg gacatttgat 1860
accgcacaga taataaaact tcttcccttc gcagccgctc caccaaagca aagtagaatg 1920
cagttctcct catttactgt gaatgtgagg ggatcaggaa tgagaatact tgtaaggggc 1980
aattctcctg tattcaacta taacaaggcc acgaagagac tcacagttct cggaaaggat 2040
gctggcactt taactgaaga cccagatgaa ggcacagctg gagtggagtc cgctgttctg 2100
aggggattcc tcattctggg caaagaagac aagagatatg ggccagcact aagcatcaat 2160
gaactgagca accttgcgaa aggagagaag gctaatgtgc taattgggca aggagacgtg 2220
gtgttggtaa tgaaacggaa acgggactct agcatactta ctgacagcca gacagcgacc 2280
aaaagaattc ggatggccat caattagtgt cgaatagttt aaaaacgacc ttgtttctac 2340
t 2341

<210> 7
<211> 1775
<212> DNA
<213> Influenza A virus

<400> 7
agcaaaagca ggggaaaata aaaacaacca aaatgaaggc aaacctactg gtcctgttat 60

```
gtgcacttgc agctgcagat gcagacacaa tatgtatagg ctaccatgcg aacaattcaa      120
ccgacactgt tgacacagta ctcgagaaga atgtgacagt gacacactct gttaacctgc      180
tcgaagacag ccacaacgga aaactatgta gattaaaagg aatagcccca ctacaattgg      240
ggaaatgtaa catcgccgga tggctcttgg gaaacccaga atgcgaccca ctgcttccag      300
tgagatcatg gtcctacatt gtagaaacac caaactctga gaatggaata tgttatccag      360
gagatttcat cgactatgag gagctgaggg agcaattgag ctcagtgtca tcattcgaaa      420
gattcgaaat atttcccaaa gaaagctcat ggcccaacca caacacaaac ggagtaacgg      480
cagcatgctc ccatgagggg aaaagcagtt tttacagaaa tttgctatgg ctgacggaga      540
aggagggctc atacccaaag ctgaaaaatt cttatgtgaa caaaaaaggg aaagaagtcc      600
ttgtactgtg ggtattcat cacccgccta acagtaagga acaacagaat ctctatcaga       660
atgaaaatgc ttatgtctct gtagtgactt caaattataa caggagattt accccggaaa      720
tagcagaaag acccaaagta agagatcaag ctgggaggat gaactattac tggaccttgc      780
taaaacccgg agacacaata atatttgagg caaatggaaa tctaatagca ccaatgtatg      840
ctttcgcact gagtagagac tttgggttcg gcatcatcac ctcaaacgca tcaatacatg      900
agtgtaacac gaagtgtcaa acacccctgg gagctataaa cagcagtctc ccttaccaga      960
atatacaccc agtcacaata ggagagtgcc caaaatacgt caggagtgcc aaattgagga     1020
tggttacagg actaaggaac attccgtcca ttcaatccag aggtctattt ggagcctttg     1080
ccggttttat tgaaggggga tggactggaa tgatagatgg atggtatggt tatcatcatc     1140
agaatgaaca gggatcaggc tatgcagcgg atcaaaaaag cacacaaaat gccattaacg     1200
gaattacaaa caaggtgaac actgttatcg agaaaatgaa cattcaattc acagctgtgg     1260
gtaaagaatt caacaaatta gaaaaaagga tggaaaattt aaataaaaaa gttgatgatg     1320
gatttctgga catttggaca tataatgcag aattgttagt tctactggaa aatgaaagga     1380
ctctggattt ccatgactca aatgtgaaga atctgtatga gaaagtaaaa agccaattaa     1440
agaataatgc caaagaaatc ggaaatagat gttttgagtt ctaccacaag tgtgacaatg     1500
aatgcatgga aagtgtaaga aatgggactt atgattatcc caaatattca gaagagtcaa     1560
agttgaacag ggaaaaggta gatggagtga aattggaatc aatggggatc tatcagattc     1620
tggcgatcta ctcaactgtc gccagttcac tggtgctttt ggtctccctg gggcaatca      1680
gtttctggat gtgttctaat ggatctttgc agtgcagaat atgcatctga gattagaatt     1740
tcagagatat gaggaaaaac acccttgttt ctact                                1775
```

<210> 8
<211> 1027
<212> DNA
<213> Influenza A virus

```
<400>  8
agcgaaagca ggtagatatt gaaagatgag tcttctaacc gaggtcgaaa cgtacgtact        60

ctctatcatc ccgtcaggcc ccctcaaagc cgagatcgca cagagacttg aagatgtctt       120

tgcagggaag aacaccgatc ttgaggttct catggaatgg ctaaagacaa gaccaatcct       180

gtcacctctg actaagggga ttttaggatt tgtgttcacg ctcaccgtgc ccagtgagcg       240

aggactgcag cgtaracgct ttgtccaaaa tgcccttaat gggaacgggg atccaaataa       300

catggacaaa gcagttaaac tgtataggaa gctcaagagg gagataacat tccatggggc       360

caaagaaatc tcactcagtt attctgctgg tgcacttgcc agttgtatgg gcctcatata       420

caacaggatg gggggctgtga ccactgaagt ggcatttggc ctggtatgtg caacctgtga      480

acagattgct gactcccagc atcggtctca taggcaaatg gttacaacaa ccaatccact       540

aatcagacat gagaacagaa tggttttagc cagcactaca gctaaggcta tggagcaaat       600

ggctgaatcg agtgagcaag cagcagaggc catggaggtt gctagtcagg ctagacaaat       660

ggtgcaagcg atgagaacca ttgggactca tcctagctcc agtgctggtc tgaaaaatga       720

tcttcttgaa aatttgcagg cctatcagaa acgaatgggg gtgcagatgc aacggttcaa       780

gtgatcctct cactattgcc gcaaatatca ttgggatctt gcacttgaca ttgtggattc       840

ttgatcgtct ttttttcaaa tgcatttacc gtcgctttaa atacggactg aaaggagggc       900

cttctacgga aggagtgcca aagtctatga gggaagaata tcgaaaggaa cagcagagtg       960

ctgtggatgc tgacgatggt cattttgtca gcatagagct ggagtaaaaa actaccttgt      1020

ttctact                                                                1027
```